(19) **Europäisches Patentamt**
**European Patent Office**
**Office européen des brevets**

(11) **EP 4 686 758 A1**

(12) **EUROPEAN PATENT APPLICATION**

(43) Date of publication:
**04.02.2026  Bulletin 2026/06**

(21) Application number: **24192194.9**

(22) Date of filing: **31.07.2024**

(51) International Patent Classification (IPC):
*C12N 15/87* (2006.01)   *C12Q 1/6806* (2018.01)

(52) Cooperative Patent Classification (CPC):
(C-Sets available)
**C12Q 1/6806; C12N 15/85;** C12N 2810/40   (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **4basebio UK Ltd**
**Cambridge CB24 5QE (GB)**

(72) Inventors:
• **WALKER, Amy**
  **Cambridge, CB24 5QE (GB)**
• **LANCKRIET, Heikki**
  **Cambridge, CB24 5QE (GB)**

• **BOUCHAREB, Amine**
  **Cambridge, CB24 5QE (GB)**
• **RISTIN, Anca**
  **Cambridge, CB24 5QE (GB)**
• **MNKANDLA, Samantha**
  **Cambridge, CB24 5QE (GB)**

(74) Representative: **Boult Wade Tennant LLP**
**Salisbury Square House**
**8 Salisbury Square**
**London EC4Y 8AP (GB)**

Remarks:
The complete document including Reference Table(s) and the Sequence Listing(s) can be downloaded from the EPO website

(54) **NUCLEIC ACID FOR NUCLEAR TRANSLOCATION**

(57)    The invention relates to a linear DNA product that is resistant to nuclease (e.g. exonuclease) digestion, and which is suitable for delivery to the nucleus of a cell.

The invention also relates to related methods of production and uses of the linear DNA product.

EP 4 686 758 A1

(52) Cooperative Patent Classification (CPC): (Cont.)

C-Sets
**C12Q 1/6806, C12Q 2525/131, C12Q 2525/125,
C12Q 2525/121, C12Q 2525/191, C12Q 2525/197,
C12Q 2525/113, C12Q 2525/203, C12Q 2525/205**

**Description**

**FIELD**

The invention relates to a linear DNA product that is resistant to nuclease (e.g. exonuclease) digestion, and which is suitable for delivery to the nucleus of a cell. The invention also relates to related methods of production and uses of the linear DNA product.

**BACKGROUND**

For effective gene therapy, it is essential for extracellular DNA to traverse the plasma membrane, navigate the cytoplasm, penetrate the nucleus, undergo transcription, and ensure the resultant mRNA is shuttled back into the cytoplasm for translation. The final steps, involving the modification and precise targeting of the synthesized protein, are critical for the therapeutic outcome. Historically, the focus has been on developing transfection agents that optimize plasma membrane targeting and entry, as well as selecting the most effective promoters. While the nuclear uptake of plasmids is significantly more effective in cells that are actively dividing, it is still possible for DNA to access the nuclei of non-dividing cells, though the extent of this occurrence appears to be essentially limited by inefficient trafficking of DNA to the nucleus.

Although nuclear entry of naked DNA in the absence of cell division is possible, it remains a challenge that has to be addressed in the gene therapy field. In the absence of mitosis and the subsequent breakdown of the nuclear envelope, the only established way for proteins and protein-DNA complexes to enter the nucleus is through nuclear pore complexes (NPCs). The NPC is an aqueous channel in the nuclear envelope through which proteins, ribonucleoproteins, and all macromolecules in the cell can traffic between the cytoplasm and nucleus. The pores are large (-125 MDa) multiprotein complexes that are composed of more than 100 distinct proteins present in multiple copies. Transport through the NPC occurs either by signal-independent diffusion in the case of small molecules such as nucleotides, ions, and solutes, or by signal-mediated facilitated diffusion.

In relation to plasmids, it was demonstrated that plasmids traffic into the nucleus through the NPC by localizing the injected DNA by in situ hybridization or directly using plasmids labeled with fluorescent peptide nucleic acid (PNA) clamps in a process that is inhibited by agents that block signal-mediated transport through the NPC (Wilson, G. Lee, et al. "Nuclear import of plasmid DNA in digitonin-permeabilized cells requires both cytoplasmic factors and specific DNA sequences." Journal of Biological Chemistry 274.31 (1999): 22025-22032).

In case of proteins, the signals needed for transport are either the NLS for nuclear import or the NES for nuclear export. RNAs, including mRNA, snRNAs, miRNAs, and tRNAs, all exist as ribonucleoprotein complexes that require both RNA and protein signals for their export or import into the nucleus. In all cases, the signals needed for nuclear import or export are recognized by a class of receptors termed the karyopherins. These fall into two general classes: importins for nuclear import and exportins for nuclear export. In the case of the importins, the NLS on a cargo protein is recognized either directly by a $\beta$ subunit (of which there are 11 in humans) with $\beta$1 being the prototype, or by an $\alpha$ subunit (7 isoforms in humans) that then binds to $\beta$1 for translocation across the NPC. Similar to importin $\alpha$, other factors can recognize motifs on RNA, such as the trimethylguanosine (m3G) cap structure of the U snRNA which is recognized by snurportin 1, and interact with importin $\beta$ subunits for complex import.

Although very little is known about the details of how DNA trafficking from and into to the nucleus occurs, some sequences or repeats such as SV40 enhancers or NFkb binding sites have been demonstrated to improve nuclear uptake of plasmid DNA (Dean et al. (1999) "Sequence requirements for plasmid nuclear import." Exp Cell Res.; and Mesika et al. (2001) "A regulated, NFkappaB-assisted import of plasmid DNA into mammalian cell nuclei." Mol Ther.).

Different strategies for plasmid-NLS translocation to the nucleus have been explored. However, the use of plasmids in the context of gene therapy is not optimal because plasmids contain segments of unwanted genetic material (e.g. antibiotic resistance genes). Similarly, in the context of gene editing, the inhibition of nuclear import of many DNA template including circular or linear ss/dsDNA remains an unsolved challenge. Thus, a need exists for a DNA product which is suitable for delivery to the nucleus and suitable for use in both gene therapy and gene editing.

**DESCRIPTION**

The inventors have developed a linear DNA product which is suitable for delivery to the nucleus of a cell (in dividing and non-dividing conditions). The linear DNA product of the present invention provides an improved DNA nucleus-delivery vehicle as compared to plasmid-based solutions due to its substantial lack of a bacterial backbone and/or antibiotic resistant genes. Lack of these features is particularly beneficial in the production of a cell delivery system, such as a viral vector or a nanoparticle, for example, for cell or gene therapy. Lack of these features makes the linear DNA product of the present invention particularly suitable for use in a pharmaceutical composition. In addition, the linear DNA product of the present invention is resistant to nuclease digestion (e.g. exonuclease digestion). The enhanced resistance to nuclease (e.g. exonuclease) digestion extends the life of the linear DNA product in a cell (e.g. the linear DNA product has enhanced resistance to intracellular exonucleases) and in a cell-free system (e.g. the linear DNA product has enhanced resistance to extracellular exonucleases). In view of this, the linear DNA product of the present invention is also particularly suitable for use in gene editing or gene therapy.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first and second adaptor molecules are nucleic acid molecules that comprise one or more nuclease (e.g. exonuclease)-resistant nucleotides; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first adaptor molecule is a nucleic acid molecule that comprises one or more nuclease (e.g. exonuclease)-resistant nucleotides, and the second adaptor molecule is a hairpin molecule; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first and second adaptor molecules are hairpin molecules; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first and second adaptor molecules are nucleic acid molecules that comprise one or more nuclease (e.g. exonuclease)-resistant nucleotides; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first adaptor molecule is a nucleic acid molecule that comprises one or more nuclease (e.g. exonuclease)-resistant nucleotides, and the second adaptor molecule is a hairpin molecule; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first and second adaptor molecules are hairpin molecules; and wherein a nuclear localisation signal (NLS) molecule is appended to the linear DNA product.

The invention also provides a linear DNA product comprising a single-stranded DNA (ssDNA) cassette; wherein the linear DNA product is resistant to nuclease digestion; and wherein a nuclear localisation signal (NLS) molecule is appended to the linear DNA product.

In all aspects and embodiments described herein, preferably, the linear DNA product is resistant to exonuclease digestion. Preferably, the linear DNA product is resistant to 3'- and 5'-exonuclease digestion.

The term "NLS molecule" as used herein is intended to encompass any molecule which improves translocation to the nucleus of a cell. In the case of the present invention, the NLS molecule improves the translocation of the linear DNA product to the nucleus of a cell (either in cell dividing or non-dividing conditions). The NLS molecule may be an NLS peptide, a DNA molecule, or an RNA aptamer. Preferably, the NLS molecule is a NLS peptide. For example, the NLS molecule may be any molecule listed in Table 1, Table 2 or Table 3.

The NLS molecule may be appended to the first adaptor molecule, the second adaptor molecule, or, if present, the third adaptor molecule, or the fourth adaptor molecule. Multiple NLS molecules may be appended to the linear DNA product. For example, at least 2, at least 3, at least 4, or at least 5 NLS molecules may be appended to the linear DNA product. If multiple NLS molecules are appended to the linear DNA product, the NLS molecules may be of the same type (e.g. NLS peptides) or of a different type (e.g. NLS peptide(s) and RNA aptamer(s)).

The first adaptor molecule and/or the second adaptor molecule may comprise at least 1, at least 2, at least 3, at least 4, or at least 5 nuclease (e.g. exonuclease)- resistant nucleotides. The first adaptor molecule and/or the second adaptor molecule may comprise at least 1, at least 2, at least 3, at least 4, or at least 5 phosphorothioated nucleotides. The third (and/or the fourth) adaptor (if present) may comprise at least 1, at least 2, at least 3, at least 4, or at least 5 nuclease (e.g. exonuclease)-resistant nucleotides. The third (and/or the fourth) adaptor (if present) may comprise at least 1, at least 2, at least 3, at least 4, or at least 5 phosphorothioated nucleotides.

The invention also provides a method of producing a linear DNA product, wherein the method comprises:

(a) contacting a double-stranded DNA molecule with an endonuclease, a ligase, a first adaptor molecule, and a second adaptor molecule to form a single contiguous aqueous volume; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the linear DNA product comprises a first adaptor molecule at a first end of the linear double-stranded region and a second adaptor molecule at a second end of the linear double-stranded region; and wherein a nuclear localisation signal (NLS) molecule is appended to the linear DNA product; and

wherein the linear DNA product is resistant to nuclease digestion.

The method may further comprise a step of amplifying a DNA template molecule comprising at least one endonuclease target sequence to generate the double-stranded DNA molecule. The DNA template molecule may be amplified by rolling circle amplification.

The method of producing a linear DNA product may comprise the steps of:

(a) amplifying a first DNA template molecule to generate a first amplified DNA product, wherein the first DNA template molecule comprises at least one endonuclease target sequence and wherein the first amplified DNA product is a first concatemer, and amplifying a second DNA template molecule to generate a second amplified DNA product, wherein the second DNA template molecule comprises at least one endonuclease target sequence and wherein the second amplified DNA product is a second concatemer;

(b) forming a single contiguous aqueous volume comprising the first amplified DNA product, the second amplified DNA product, an endonuclease, a ligase, a first adaptor molecule, a second adaptor molecule and a third adaptor molecule; and

(c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product; and wherein the NLS molecule is appended to the linear DNA product.

The first adaptor molecule may be at a first end of the linear double-stranded region. The second adaptor molecule may be at a second end of the linear double-stranded region. The third adaptor may (i) separate the linear portion of the first amplified DNA product from the linear portion of the second amplified product; (ii) be between the first adaptor molecule and the first end of the linear double-stranded region; or (iii) be between the second end of the linear double-stranded region and the second adaptor molecule.

The invention also provides the use of the linear DNA product as described herein for nuclear expression of a gene of interest.

The invention also provides the use of the linear DNA product as described herein in gene editing.

The invention also provides the use of the linear DNA product as described herein in gene therapy.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

## 1. Linear DNA product

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first and second adaptor molecules are nucleic acid molecules that comprise one or more nuclease (e.g. exonuclease)-resistant nucleotides; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first adaptor molecule is a nucleic acid molecule that comprises one or more nuclease (e.g. exonuclease)-resistant nucleotides, and the second adaptor molecule is a hairpin molecule; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region; and
c) a second adaptor molecule at a second end of the linear double-stranded region;

wherein the linear DNA product is resistant to nuclease digestion; wherein the first and second adaptor molecules are hairpin molecules; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first and second adaptor molecules are nucleic acid molecules that comprise one or more nuclease (e.g. exonuclease)-resistant nucleotides; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first adaptor molecule is a nucleic acid molecule that comprises one or more nuclease (e.g. exonuclease)-resistant nucleotides, and the second adaptor molecule is a hairpin molecule; and wherein a nuclear localisation signal (NLS) molecule is appended to the linear DNA product.

The invention provides a linear deoxyribonucleic acid (DNA) product comprising:

a) a linear double-stranded region;
b) a first adaptor molecule at a first end of the linear double-stranded region;
c) a second adaptor molecule at a second end of the linear double-stranded region; and
d) a third adaptor molecule which (i) separates two sequences (i.e. two cassette sequences) of the linear double-stranded region; (ii) is between the first adaptor molecule and the first end of the linear double-stranded region; or (iii) is between the second end of the linear double-stranded region and the second adaptor molecule;

wherein the linear DNA product is resistant to nuclease digestion; wherein the first and second adaptor molecules are hairpin molecules; and wherein a nuclear localisation signal (NLS) molecule is appended to the linear DNA product.

The invention also provides a linear DNA product comprising a single-stranded DNA (ssDNA) cassette; wherein the linear DNA product is resistant to nuclease digestion; and wherein a nuclear localisation signal (NLS) molecule is appended to the linear DNA product.

The term "NLS molecule" as used herein is intended to encompass any molecule which improves translocation to the nucleus of a cell. In the case of the present invention, the NLS molecule improves the translocation of the linear DNA product to the nucleus of a cell (either in cell dividing or non-dividing conditions). The expression "improves translocation" as used herein may refer to improved rate of translocation to the nucleus as compared to the rate of translocation to the nucleus without the NLS molecules being appended to the linear DNA product. The expression "improves translocation" as used herein may refer to higher quantity of the linear DNA product translocated to the nucleus as compared to the quantity of the linear DNA product translocated to the nucleus without the NLS molecule(s) being appended to the linear DNA product. For example, the improvement may be by at least 10%, at least 20%, least 30%, at least 40%, least 50%, at least 60%, least 70%, at least 80%, least 90%, or at least 100%. That is to say that at least 10%, at least 20%, least 30%, at least 40%, least 50%, at least 60%, least 70%, at least 80%, least 90%, or at least 100% more of linear DNA products is translocated to the nucleus. For example, the NLS molecule appended to the linear DNA product may result in at least 2x, at least 3x, at least 4x, at least 5x, at least 6x, at least 7x, or at least 8x more product being translocated to the nucleus. For example, the NLS molecule appended to the linear DNA product may result in at least 2x, at least 3x, at least 4x, at least 5x, at least 6x, at least 7x, or at least 8x more transcription of the linear DNA product in the nucleus. The skilled person knows different ways of measuring the quantity of the linear DNA product in the nucleus of a cell. For example, the signal corresponding to the gene expressed by the linear DNA product can be measured. For example, if the linear DNA product encodes GFP protein, a fluorescent signal of the GFP protein can be measured.

The NLS molecule may be an NLS peptide, a DNA molecule, or an RNA aptamer. Preferably, the NLS molecule is a NLS peptide. For example, the NLS molecule may be a molecule listed in Table 1, Table 2 or Table 3. The NLS molecule may interact with an importin $\alpha/\beta1$ receptor. The NLS molecule may interact with an importin $\beta_s$ receptor. The NLS molecule may interact with an importin $\alpha_s/\beta_s$ receptor. The NLS molecule may have a sequence of 90% identity with either one of SEQ ID NOs: 1-22 or 28. The NLS molecule may have a sequence of either one of SEQ ID NOs: 1-22 or 28. The NLS molecule may have a sequence of 90% identity with either one of SEQ ID NOs: 23-24. The NLS molecule may have a sequence of either one of SEQ ID NOs: 23-24. The NLS molecule may have a sequence of 90% identity with SEQ ID NOs: 27. The NLS molecule may have a sequence of SEQ ID NOs: 27. The NLS molecule may comprise a SV40 enhancer sequence or a part thereof. The NLS molecule may comprise a NF-$\kappa$B binding site sequence or a part thereof. The NLS molecule may additionally comprise a cysteine amino acid.

Table 1. Examples of peptide-based NLS molecules.

| Category | | Source | Sequence | SE Q ID NO: | Transpo rt receptor s |
|---|---|---|---|---|---|
| Classical nuclear localizatio n sig-nals (cNLS) | MP NLS | VACM-1/CUL5 | PKLKRQ | 1 | Importin α/β1 |
| | | CXCR4 | RPRK | 2 | |
| | | VP1 | RRARRPRG | 3 | |
| | BP NLS | 53BP1 | GKRKLITSEEERSPAKRGRKS | 4 | |
| | | ING4 | KGKKGRTQKEKKAARARSKGKN | 5 | |
| | | IER5 | RKRCAAGVGGGPAGCPAPGSTPLKKPRR | 6 | |
| | | ERK5 | RKPVTAQERQREREEKRRRRQERAKEREKRRQER ER | 7 | |
| Non-classical nu-clear localizatio n signals (ncNLS) | PY-NLS | Hrp1 | RSGGNHRRNGRGGRGGYNRRNNGYHPY | 8 | Importin βs |
| | | UL79 | TLLLRETMNNLGVSDHAVLSRKTPQPY | 9 | |
| | | EWS | PGKMDKGEHRQERRDRPY | 10 | |
| | Other non-classic al NLS | PTHrP | GKKKKGKPGKRREQRKKKRRT | 11 | |
| | | Pho4 | SANKVTKNKSNSSPYLNKRKGKPGPDS | 12 | |
| | | rpL23a | VHSHKKKKIPTSPTFTTPKTLTLRRQPKYPRKSAPRRNK LDHY | 13 | |

(continued)

| Category | | Source | Sequence | SE Q ID NO: | Transpo rt receptor s |
|---|---|---|---|---|---|
| Other types of nuclear localizatio n signals | Putativ e NLS | PABPN1 | None | | Importin $\alpha_s/\beta_s$ |
| | Spatial epitope NLS | STAT1 | None | | |
| | Cryptic NLS | FGF2 | None | | |
| | Multipl e NLS | MSX1 | RKHKTNRKPR | 14 | |
| | | | NRRAKAKR | 15 | |
| | | NLS-RAR$\alpha$ | RNKKKK | 16 | |
| | | | RKVIK | 17 | |
| | | SV40 Large T-antigen | PKKKRKV | 18 | |
| | | c-Myc | PAAKRVKLD | 19 | |
| | | Nucleoplasmi n | KRPAATKKAGQAKKKK | 20 | |
| | | Simian SV40 | PKKKRKV | 18 | |
| | | HIV-1 | TRQARRNRRRWRERQ | 21 | |
| | | SV40 | PKKKRKVEDPYC | 22 | |

Table 2. Examples of DNA-based NLS molecules. SEQ ID NOs: 23 and 24 comprise some repeated sequences. In bold is the sequence repeated 5x for SEQ ID NO: 23 and 2x for SEQ ID NO: 24.

| Category | Source | Sequence | SEQ ID NO: |
|---|---|---|---|
| DNA sequence | NF-κB (Binding sites) | **GGGGACTTTC**CGGGGACTTTCCGGGGACTTTCCGG GGACTTTCCGGGGACTTTCC | 23 |
| | SV40 Enhancer (Binding sites) | **TGGTTGCTGACTAATTGAGATGCATGCTTTGCATAC TTCTGCCTGCTGGGGAGCCTGGGGACTTTCCACAC C**TGGTTGCTGACTAATTGAGATGCATGCTTTGCATAC TTCTGCCTGCTGGGGAGCCTGGGGACTTTCCACACC | 24 |

Table 3. Example of RNA aptamer-based NLS molecule. The nucleotides in bold denote the RNA part of the RNA/ DNA hybrid.

| Category | Sequence | SEQ ID NO: |
|---|---|---|
| RNA aptamer | agggctaacatttg**gauccugagcuacugacuuugggucgaauugguuggcucgcccucu ucuuggaauucaggagggcgauugaacggacaccacuacugaccauacac**caaatgttag | 27 |

The NLS molecule may be appended to the first adaptor molecule, the second adaptor molecule, or, if present, the third (and/or the fourth) adaptor molecule. Multiple NLS molecules may be appended to the linear DNA product. For example, at least 2, at least 3, at least 4, or at least 5 NLS molecules may be appended to the linear DNA product. If multiple NLS molecules are appended to the linear DNA product, the NLS molecules may be of the same type (e.g. NLS peptides) or of a different type (e.g. NLS peptide(s) and RNA aptamer(s)). If multiple NLS molecules are appended to the linear DNA product, the first NLS molecule may be appended to the first adaptor molecule and the second NLS molecule may be appended to the second adaptor molecule. The first NLS molecule may be appended to the first strand of the first adaptor molecule, the second NLS molecule may be appended to the first strand of the second adaptor molecule, the third NLS molecule may be appended to the second strand of the first adaptor molecule, and the fourth NLS molecule may be appended to the second strand of the second adaptor molecule. Multiple NLS molecules may be appended to the first, second, and/or (if present) the third (and/or the fourth) adaptor molecule(s). Multiple NLS molecules may be appended to each strand of the first, second, and/or (if present) the third (and/or the fourth) adaptor molecule(s). That is to say that at least 2, at least 3, at least 4, or at least 5 NLS molecules may be appended to the first, second, and/or (if present) the third (and/or the fourth) adaptor molecule(s). At least 2, at least 3, at least 4, or at least 5 NLS molecules may be appended to each strand of the first, second, and/or (if present) the third (and/or the fourth) adaptor molecule(s). Preferably, at least one NLS molecule is appended to each one of: (i) the first adaptor molecule; and (ii) the second adaptor molecule. At least one NLS molecule may be appended to each strand of the first and the second adaptor molecule. Preferably, at least one NLS peptide is appended to each one of: (i) the first adaptor molecule; and (ii) the second adaptor molecule. At least one NLS peptide may be appended to each strand of the first and the second adaptor molecule. That is to say that, preferably, at least two NLS molecules (e.g. NLS peptides) are appended to a linear DNA product.

One of the at least two NLS molecules may be appended to the first adaptor molecule and the other of the at least two NLS molecules may be appended to the second adaptor molecule. One of the at least two NLS molecules may be appended to the first strand of the first adaptor molecule and the other of the at least two NLS molecules may be appended to the second strand of the first adaptor molecule.

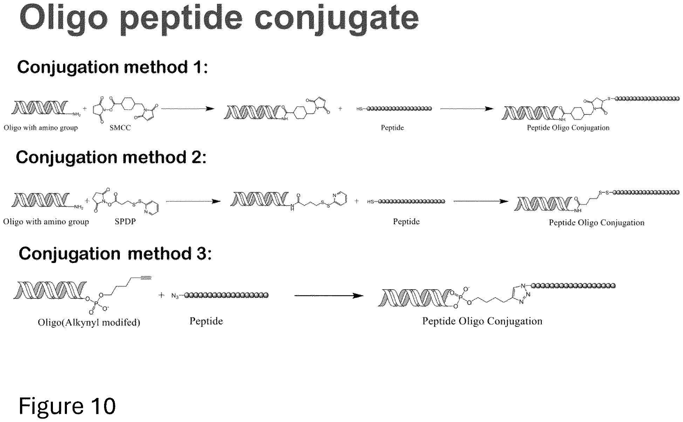

The term "appended" as used herein in the context of appending the NLS molecule to the linear DNA product is intended to encompass any suitable attachment methods. For example, if the NLS molecule is a DNA molecule, the NLS molecule may be ligated or annealed to the linear DNA product. For example, if the NLS molecule is an NLS peptide, the NLS molecule may be conjugated to the linear DNA product. The NLS molecule may be appended to the linear DNA product using a linker. The linker may comprise N-hydroxysuccinimide (NHS) ester, a maleimide group, and/or carbonyldiimidazole. The linker may be 4-(N-maleimidomethyl)-cyclohexane-1-carboxylic acid N-hydroxysuccinimide ester (SMCC). Different conjugation methods are known to the skilled person. For example, suitable conjugation methods are illustrated in Figure 10. In one example, the NLS molecule may be conjugated to the linear DNA product using a SMCC linker or a succinimidyl 3-(2-pyridyldithio)propionate (SPDP) linker. Alternatively, the NLS molecule may be appended to the linear DNA product directly (i.e. without a linker). In this case, the linear DNA product may comprise a modified nucleotide to allow the binding of the NLS molecule. For example, the linear DNA product may comprise an alkynyl modified oligonucleotide. The NLS molecule (e.g. the NLS peptide) may comprise an additional residue to allow for the appending to the linear DNA product. For example, the NLS molecule (e.g. the NLS peptide) may comprise an additional cysteine amino acid. The cysteine amino acid may be at the start (i.e. N-terminal end) of the NLS peptide sequence.

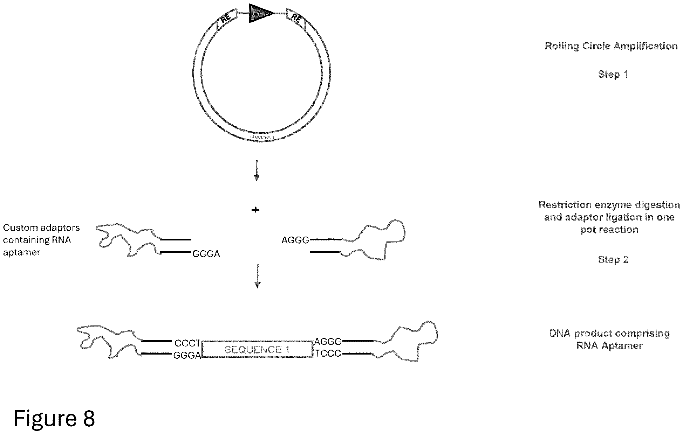

In the embodiments where the NLS molecule is an RNA aptamer (such as the embodiment in Figure 8), the RNA aptamer may form a hairpin. In this embodiment, a first RNA aptamer may append to the first strand of the first adaptor molecule and to the second strand of the first adaptor molecule to form a hairpin structure. Similarly, a second RNA aptamer may append to the first strand of the second adaptor molecule and to the second strand of the second adaptor molecule to form a hairpin structure. That is to say that each end of the RNA aptamer appends to one of the strands of the first and/or second adaptor molecule. Thus, the linear DNA product may be closed at each end by an RNA aptamer. In this embodiment, preferably the first adaptor and/or the second adaptor molecule comprise at least one nuclease (e.g. exonuclease)- resistant nucleotide. Preferably, both strands of the first adaptor and/or the second adaptor molecule comprise at least one nuclease (e.g. exonuclease)- resistant nucleotide.

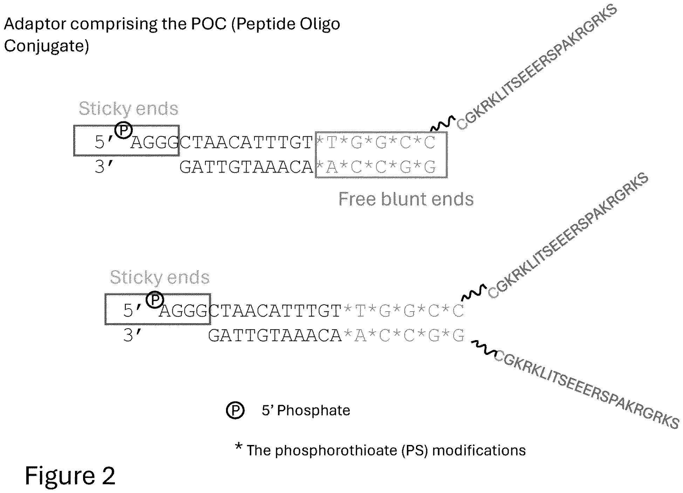

In the embodiments where the NLS molecule is an NLS peptide (such as the embodiment in Figure 2), the NLS peptide may append to the first strand and/or the second strand of the first adaptor molecule. Additionally or alternatively, the NLS peptide may append to the first strand and/or the second strand of the second adaptor molecule. In this embodiment, preferably the first adaptor and/or the second adaptor molecule comprise at least one nuclease (e.g. exonuclease)- resistant nucleotide. Preferably, both strands of the first adaptor and/or the second adaptor molecule comprise at least one nuclease (e.g. exonuclease)- resistant nucleotide.

The first adaptor molecule and/or the second adaptor molecule may comprise at least 1, at least 2, at least 3, at least 4, or at least 5 nuclease (e.g. exonuclease)- resistant nucleotides. The first adaptor molecule and/or the second adaptor molecule may comprise at least 1, at least 2, at least 3, at least 4, or at least 5 phosphorothioated nucleotides. The third adaptor (if present) may comprise at least 1, at least 2, at least 3, at least 4, or at least 5 nuclease (e.g. exonuclease)- resistant nucleotides. The third adaptor (if present) may comprise at least 1, at least 2, at least 3, at least 4, or at least 5 phosphorothioated nucleotides.

In the embodiments wherein the linear DNA product comprises a ssDNA cassette, the linear DNA product may comprise a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette. The nuclease-resistant nucleotides may be incorporated into the linear DNA product in the form of a first adaptor molecule and/or a second adaptor molecule. The NLS molecule may be located at the 5' end of the ssDNA cassette or 5' of the ssDNA cassette and/or the 3' end of the ssDNA cassette or 3' of the ssDNA cassette,

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease digestion). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The linear DNA product may have enhanced resistance to nuclease digestion (e.g. exonuclease digestion). The enhanced resistance to nuclease (e.g. exonuclease) digestion may extend the life of the linear DNA product in a cell (i.e. the linear DNA product has enhanced resistance to intracellular exonucleases) and in a cell-free system (i.e. the linear DNA product has enhanced resistance to extracellular exonucleases). The enhanced resistance to exonuclease digestion may be provided by appending (e.g. ligating) a first adaptor molecule to a first end of the linear double-stranded region and a second adaptor to a second end of the linear double stranded region. In this regard, the entire content of WO2023/006978 A1 is incorporated herein by reference.

The first adaptor molecule and the second adaptor molecule may both be linear adaptor molecules comprising nuclease resistant nucleotides (i.e. forming a linear open-ended DNA product).

The first adaptor molecule and the second adaptor molecule may each comprise a hairpin or a stem-loop (i.e. forming a closed linear DNA product).

The first adaptor molecule may be a linear adaptor molecule comprising nuclease resistant nucleotides and the second adaptor molecule may comprise a hairpin or a stem-loop (or vice versa), (i.e. forming a partially closed linear DNA product). The partially closed linear DNA product may be a partially covalently closed DNA product.

The adaptor molecules may provide protection from digestion by exonucleases that cleave the 3'-end nucleotides (e.g. exonuclease III) and exonucleases that cleave the 5'-end nucleotides (e.g. exonuclease VIII). Thus, the linear DNA product may be resistant to exonuclease digestion. Preferably, the linear DNA product is resistant to 3'- and 5'-exonuclease digestion.

The first and second adaptor molecules may be identical molecules, or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. The linear DNA product produced by the methods described herein may be resistant to nuclease (e.g. exonuclease) digestion.

The first adaptor molecule may be a nucleic acid adaptor molecule (e.g. DNA). The second adaptor molecule may be a nucleic acid adaptor molecule (e.g. DNA). The first adaptor molecule and/or the second adaptor molecule may be a synthetic adaptor molecule. The first adaptor molecule and/or the second adaptor molecule may not be a plasmid or a vector DNA.

The first adaptor molecule and/or the second adaptor molecule may comprise a modified nucleotide (e.g. an alkynyl modified nucleotide) to enable the appending of an NLS molecule. The first adaptor molecule and/or the second adaptor molecule may comprise at least 2, at least 3, at least 4, or at least 5 modified nucleotides (e.g. alkynyl modified nucleotides) to enable the appending of at least 2, at least 3, at least 4, or at least 5 NLS molecules.

The first adaptor molecule and/or the second adaptor molecule may comprise an amino group (i.e. - $NH_2$) to enable the appending of an NLS molecule. The first adaptor molecule and/or the second adaptor molecule may comprise at least 2, at least 3, at least 4, or at least 5 amino groups (i.e. -$NH_2$) to enable the appending of at least 2, at least 3, at least 4, or at least 5 NLS molecules.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end. The overhang may have at least 3 nucleotides (preferably from 4 to 6 nucleotides). The overhang of the first adaptor molecule and/or the second adaptor molecule may be complementary to the first and/or second end of the linear double-stranded region. The overhang of the first adaptor molecule and/or the second adaptor molecule may anneal to the first and/or second end of the linear double-stranded region.

The first adaptor molecule and/or the second adaptor molecule may comprise a self-complementary element which creates a loop, such as a hairpin loop or a stem- loop. That is to say that the adaptor may initially be provided in a linear form, and in the methods described herein may fold on itself to form a hairpin loop or a stem-loop. The first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region may comprise a 3' or a 5' overhang.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may be at least 5, at least 10, at least 12, at least 14, at least 15, at least 16, at least 18, at least 20, at least 22, at least 24, at least 26, at least 28, at least 30, at least 32, at least 34, at least 36, at least 38, or at least 40 nucleotides. Preferably, the single-stranded portion is at least 10, at least 12 or at least 14 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs. Preferably, the double-stranded portion is at least 10 base pairs long.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first adaptor molecule may comprise a portion (e.g. the overhang) that is complementary to the first end of the linear double-stranded region. The second adaptor molecule may comprise a portion (e.g. the overhang) that is complementary to the second end of the linear double-stranded region. The first adaptor molecule may comprise a portion that anneals to the first end of the linear double-stranded region. The second adaptor molecule may comprise a portion that anneals to the second end of the linear double-stranded region. The first adaptor molecule may comprise a portion that is complementary and anneals to the first end of the linear double-stranded region. The second adaptor molecule may comprise a portion that is complementary and anneals to the second end of the linear double-stranded region.

The portion that is complementary or anneals to the first or second end of the linear double-stranded region may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the linear double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may not comprise a Type IIS endonuclease target sequence. The first adaptor molecule and/or the second adaptor molecule may not comprise BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequences.

The first adaptor molecule and/or the second adaptor molecule may comprise one or more locked nucleic acids (LNAs).

The first adaptor molecule and/or the second adaptor molecule may comprise one or more nuclease-resistant nucleotides (i.e. protected nucleotides), such as phosphorothioated nucleotides. The nuclease-resistant nucleotides may be located in the single-stranded portion (e.g. hairpin portion) or the double-stranded portion of the adaptor molecules . The nuclease-resistant nucleotides may be located in the overhang portion of the adaptor molecules.

The first adaptor molecule and/or the second adaptor molecule may confer resistance to 3'-end exonuclease digestion (e.g. by exonuclease III) and/or 5'-end exonuclease digestion (e.g. by exonuclease VIII).

The closing of the linear double-stranded region at the first end may generate a first closed end of the closed linear DNA product. The closing of the linear double-stranded region at the second end may generate a second closed end of the closed linear DNA product. The first closed end and the second closed end of the closed linear DNA product may be resistant to nuclease digestion. The nuclease digestion may be exonuclease digestion. Preferably, the first closed end and the second closed end of the closed linear DNA product confer resistance to 3'-end exonuclease digestion (e.g. by exonuclease III) and/or 5'-end exonuclease digestion (e.g. by exonuclease VIII).

The first and second adaptor molecules may comprise one or more nuclease-resistant nucleotides (e.g. phosphorothioated nucleotides), such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

Each adaptor molecule may comprise a plurality of phosphorothioated nucleotides. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may be double-stranded (e.g. double-stranded DNA). Each adaptor molecule may comprise a portion that is double-stranded (e.g. double-stranded DNA).

The first and/or second adaptor molecules may each comprise at least 3, at least 4, at least 5, at least 6, at least 7, at least 8, at least 9, at least 10, at least 11, at least 12, at least 13, at least 14, at least 15, or at least 16 base pairs.

Each adaptor molecule may comprise a plurality of phosphorothioated nucleotides in each strand. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand. Preferably, the adaptor molecule comprises at least 5 phosphorothioated nucleotides. Preferably, the adaptor molecule comprises at least 5 phosphorothioated nucleotides in each strand.

Each adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

The nuclease-resistant nucleotides (i.e. nucleotides resistant to exonuclease digestion) may be phosphorothioated nucleotides of at least one type. For example, the at least one type of phosphorothioated nucleotides is $\alpha$-S-dATP (i.e. 2'-deoxyadenosine-5'-($\alpha$-thio)-triphosphate), $\alpha$-S-dCTP (i.e. 2'-deoxycytidine-5'-($\alpha$-thio)-triphosphate), $\alpha$-S-dGTP (i.e. 2'-deoxyguanosine-5'-($\alpha$-thio)-triphosphate), $\alpha$-S-dTTP (i.e. 2'-deoxythymidine-5'-($\alpha$-thio)-triphosphate), $\alpha$-S-dUTP (i.e. 2'-deoxyuridine-5'-($\alpha$-thio)-triphosphate), and/or uridine 2', 3'-cyclophosphorothioate.

The adaptor molecules may comprise at least two types of phosphorothioated nucleotides. For example, the at least two types of phosphorothioated nucleotides are: $\alpha$-S-dATP and $\alpha$-S-dCTP, $\alpha$-S-dATP and $\alpha$-S-dGTP, $\alpha$-S-dATP and $\alpha$-S-dTTP, $\alpha$-S-dCTP and $\alpha$-S-dGTP, $\alpha$-S-dCTP and $\alpha$-S-dTTP, or $\alpha$-S-dGTP and $\alpha$-S-dTTP.

The adaptor molecules may comprise at least three types of phosphorothioated nucleotides. For example, the at least three types of phosphorothioated nucleotides are:

(a) $\alpha$-S-dATP, $\alpha$-S-dCTP and $\alpha$-S-dGTP;
(b) $\alpha$-S-dATP, $\alpha$-S-dCTP and $\alpha$-S-dTTP;
(c) $\alpha$-S-dATP, $\alpha$-S-dGTP and $\alpha$-S-dTTP; or
(d) $\alpha$-S-dCTP, $\alpha$-S-dGTP and $\alpha$-S-dTTP.

The adaptor molecules may comprise at least four types of phosphorothioated nucleotides. For example, the at least four types of nuclease-resistant nucleotides are $\alpha$-S-dATP, $\alpha$-S-dCTP, $\alpha$-S-dGTP and $\alpha$-S-dTTP.

The nuclease-resistant nucleotides (i.e. nucleotides resistant to exonuclease digestion) may be 2'-O-methyl nucleotides or 2'-O-methoxyethyl (MOE) nucleotides. The nuclease-resistant nucleotides may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, an RNA sequence or a DNA/RNA chimera sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enables the detection of the double-stranded nucleic acid molecule via, physical, chemical and/or biological means. The label may be a chromophore, a fluorophore and/or a radioactive molecule.

To facilitate detection and/or quantification of the DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeqTM, MiSeqTM and/or Genome AnalyzerTM sequencing systems), Oxford NanoporeTM Technologies (e.g. the MinION sequencing system), Ion TorrentTM (e.g. the Ion PGMTM and/or Ion ProtonTM sequencing systems), Pacific Biosciences (e.g. the PACBIO RS II sequencing system); Life TechnologiesTM (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different serotypes. An inverted terminal repeat sequence may comprise a terminal resolution site and a Rep binding site.

The first adaptor molecule and/or the second adaptor molecule may comprise an aptamer.

The expression "at a first end" or "at a second end" of the linear double-stranded region as used herein in the context of the first adaptor molecule and/or the second adaptor molecule means that the adaptors are attached to the ends of the linear double-stranded region. The first end may be the 5' end of the linear double-stranded region. The second end may be the 3' end of the linear double-stranded region. The adaptors may be attached to the linear double-stranded region by annealing complementary nucleotides (e.g. if the adaptor has an overhang), and/or by ligation. The adaptors and/or the linear double-stranded region may be pre-processed by digesting (with an endonuclease) the restriction enzyme sites to allow annealing and/or ligation of the adaptors to the linear double-stranded region.

The linear DNA product may comprise a third adaptor molecule. The linear DNA product may comprise n adaptor molecules, wherein n is between 1-100.

The above description (i.e. embodiments and examples of the first adaptor molecule and the second adaptor molecule) apply *mutatis mutandis* to the third adaptor molecule (if present). The above description (i.e. embodiments and examples of the first adaptor molecule and the second adaptor molecule) apply *mutatis mutandis* to the n adaptor molecules (if present).

The third adaptor molecule may separate two sequences (i.e. two cassette sequences) of the linear double-stranded region. The two sequences (i.e. two cassette sequences) of the linear double-stranded region may be the same or different.

The third adaptor molecule may be located between the first adaptor molecule and the first end of the linear double-stranded region. The third adaptor molecule may be located between the second end of the linear double-stranded region and the second adaptor molecule.

The third adaptor molecule may comprise at least 3, at least 4, at least 5, at least 6, at least 7, at least 8, at least 9, at least 10, at least 12, at least 14, at least 16, at least 18, at least 20, at least 22, at least 24, at least 26, at least 28, or at least 30 nucleotides (or base pairs, if the adaptor is double-stranded). Preferably, the third adaptor molecule is a double-stranded linear DNA molecule. Unlike the first adaptor molecule and the second adaptor molecule, the function of the third adaptor is not typically to provide resistance to nuclease digestion (because the third adaptor molecule is not typically located at the first end or the second end of the linear DNA product). Nonetheless, the third adaptor molecule may comprise nuclease-resistant nucleotides. The function of the third adaptor molecule may be to provide means for appending a functional portion. The function of the third adaptor molecule may be to provide means for appending a NLS molecule (or multiple NLS molecules).

The linear DNA product may comprise a fourth adaptor molecule.

The above description (i.e. embodiments and examples of the first adaptor molecule and the second adaptor molecule) apply *mutatis mutandis* to the fourth adaptor molecule (if present).

The fourth adaptor molecule may separate two sequences (i.e. two cassette sequences) of the linear double-stranded region. The two sequences (i.e. two cassette sequences) of the linear double-stranded region may be the same or different.

The fourth adaptor molecule may be located between the first adaptor molecule and the first end of the linear double-stranded region. The fourth adaptor molecule may be located between the second end of the linear double-stranded region and the second adaptor molecule.

The fourth adaptor molecule may comprise at least 3, at least 4, at least 5, at least 6, at least 7, at least 8, at least 9, at least 10, at least 12, at least 14, at least 16, at least 18, at least 20, at least 22, at least 24, at least 26, at least 28, or at least 30 nucleotides (or base pairs, if the adaptor is double-stranded). Preferably, the fourth adaptor molecule is a double-stranded linear DNA molecule. Unlike the first adaptor molecule and the second adaptor molecule, the function of the fourth adaptor is not typically to provide resistance to nuclease digestion (because the fourth adaptor molecule is not typically located at the first end or the second end of the linear DNA product). Nonetheless, the fourth adaptor molecule may comprise nuclease-resistant nucleotides. The function of the fourth adaptor molecule may be to provide means for appending a functional portion. The function of the fourth adaptor molecule may be to provide means for appending a NLS molecule (or multiple NLS molecules).

The linear DNA product may comprise a third adaptor molecule and a fourth adaptor molecule. The third adaptor molecule and the fourth adaptor molecule may each function as means for appending of at least one NLS molecule (e.g. at least one NLS peptide). If the linear DNA product comprises the third and the fourth adaptor molecules, the third adaptor molecule may, for example, separate two sequences (i.e. two cassette sequences) of the linear double-stranded region and the fourth adaptor may, for example, be located between the first adaptor molecule and the first end of the linear double-stranded region. The third adaptor molecule may, for example, be located between the second end of the linear double-stranded region and the second adaptor; and the fourth adaptor may, for example, be located between the first adaptor molecule and the first end of the linear double-stranded region.

The third adaptor molecule and/or the fourth adaptor molecule (and/or the n adaptor molecules) may be incorporated into the linear DNA product by the methods described herein.

As discussed herein, different types of adaptor molecules may be appended to the linear double-stranded region. Depending on the type of adaptor molecules used, the linear DNA product of the present invention may be an open-ended linear DNA product, a partially closed linear DNA product or a closed linear DNA product. In addition, the open-ended linear DNA product may be closed or partially closed by the NLS molecule (e.g. an RNA aptamer).

The linear DNA product may be an open-ended linear DNA product, optionally wherein the linear DNA product comprises a first adaptor molecule at a first end and a second adaptor molecule at a second end. Preferably, the first and the second adaptor molecules each comprise at least one nuclease resistant nucleotide.

The linear DNA product may be a closed linear DNA product, optionally wherein the linear double-stranded region is closed at a first end by a first adaptor molecule and closed at a second end by a second adaptor molecule. Preferably, wherein the first and the second adaptor molecules are each hairpin molecules. The linear double-stranded region may be closed at a first end by protelomerase (e.g. TelN) or closed at a second end by protelomerase (e.g. TelN), optionally wherein a first adaptor molecule comprises a protelomerase target sequence or a portion thereof and/or a second adaptor molecule comprises a protelomerase target sequence or a portion thereof. The linear double-stranded region may be closed at a first end by a first adaptor molecule and closed at a second end by protelomerase (e.g. TelN) (optionally wherein a second adaptor molecule comprises a protelomerase target sequence or a portion thereof). The linear double-stranded region may be closed at a first end by protelomerase (optionally wherein a first adaptor molecule comprises a protelomerase target sequence or a portion thereof) and closed at a second end by a second adaptor molecule.

The linear double-stranded region may be a partially closed linear DNA, optionally wherein the linear double-stranded region is closed at a first end by a first adaptor molecule or closed at a second end by a second adaptor molecule. Preferably, wherein the first adaptor molecule or the second adaptor molecule is a hairpin molecule. The partially closed linear double-stranded region may be closed at a first end by protelomerase (e.g. TelN), optionally wherein a first adaptor molecule comprises a protelomerase target sequence or a portion thereof. The partially closed linear double-stranded region may be closed at a second end by protelomerase (e.g. TelN), optionally wherein a second adaptor molecule comprises a protelomerase target sequence or a portion thereof.

The linear DNA product may comprise a DNA cassette. The linear double-stranded region of the linear DNA product may comprise a DNA cassette. In some embodiments, the DNA cassette may be difficult to completely and/or faithfully amplify from a single DNA template molecule. DNA cassettes may be difficult to completely and/or faithfully amplify from a single DNA template molecule due to their size and/or sequence composition (e.g. high GC content and/or presence of repeat regions such as palindromic repeats). In those embodiments, a part of the DNA cassette may be provided by the first adaptor molecule and/or the second adaptor molecule. The DNA cassette may be formed of at least 3, at least 4, at least 5, at least 6, at least 7, at least 8, at least 9 or at least 10 cassette sequences. The DNA cassette may be formed of n cassette sequences, wherein n is between 1-100, preferably, between 1-10. The cassette sequences may be separated with an adaptor molecule (e.g. a third or fourth adaptor molecule as described herein). The cassette sequences may be separated with an adaptor molecule (e.g. a third or fourth adaptor molecule as described herein) which is appended to an NLS molecule.

The linear DNA product may comprise a single-stranded DNA cassette or a double-stranded DNA cassette.

Each of the cassette sequences may provide a different sequence of the DNA cassette. Alternatively, at least 2, at least 3, at least 4, at least 5, at least 6, at least 7, at least 8, at least 9 or at least 10 cassette sequences, may provide the same sequence of the DNA cassette. The DNA cassette may be formed of at least 2, at least 3, at least 4, at least 5, at least 6, at least 7, at least 8, at least 9, or at least 10 repeats of the same sequence.

The skilled person would be aware of a range of options for the composition of the DNA cassette and sequence components that might be included in such a cassette. Various non-limiting options for the cassette are described herein.

The DNA cassette may be at least 1, at least 2, at least 3, at least 4, at least 5, at least 6, at least 7, at least 8, at least 9, at least 10, at least 11, at least 12, at least 13, at least 14 or at least 15 kilobases (kb) in length. Thus, the linear double-stranded portion may be at least 1, at least 2, at least 3, at least 4, at least 5, at least 6, at least 7, at least 8, at least 9, at least 10, at least 11, at least 12, at least 13, at least 14, at least 15, at least 20, or at least 30 kilobases (kb) in length. In the embodiments in which the DNA cassette is single-stranded, the DNA cassette may be at least 1, at least 2, at least 3, at least 4, at least 5, at least 6, at least 7, at least 8, at least 9, at least 10, at least 11, at least 12, at least 13, at least 14 or at least 15 kilobases (kb) in length. Thus, the linear double-stranded portion may be at least 1, at least 2, at least 3, at least 4, at least 5, at least 6, at least 7, at least 8, at least 9, at least 10, at least 11, at least 12, at least 13, at least 14, at least 15, at least 20, or at least 30 thousands nucleotides in length.

The DNA cassette (or a cassette sequence) may comprise a GC content of at least 50%, at least 60%, at least 65%, at least 70%, at least 75%, at least 80%, at least 85%, at least 90%, at least 95%, at least 100%. Preferably, the DNA cassette (or a cassette sequence) may have a GC content of at least 60%. More preferably, the DNA cassette (or a cassette sequence) may have a GC content of at least 65%. GC content may refer to the percentage of bases that are either guanine or cytosine.

The DNA cassette (or a cassette sequence) may comprise a repeated sequence. Repeated sequence may refer to a sequence in which two or more identical or extremely similar nucleotide sequences are repeated. A repeated sequence includes but is not limited to a tandem repeat or interspersed repeat. The repeated sequence may be a direct repeat or inverted repeat (e.g. a palindromic repeat). Tandem repeats are repeated sequences which are directly adjacent to each other. Interspersed repeats are repeated sequences which are found in different locations. Direct repeats occur when a nucleotide sequence is repeated with the same directionality. Inverted repeats occur when a nucleotide sequence is repeated in the inverse direction. When there are no nucleotides separating the inverted repeat, the sequence is called a palindromic repeat. The repeated sequence may be at least 2, 5, 10, 25, 50, 75, 100, 250, 500 or 750 nucleotides long. Preferably, the repeated sequence is at least 100 nucleotides long. The repeated sequence may be 2-1000 nucleotides long, 3-500 nucleotides long, 4-250 nucleotides long, 5-100 nucleotides long, 10-1000 nucleotides long, 15-500 nucleotides long, 20-250 nucleotides long, 25-100 nucleotides long. Preferably, the repeated sequence is 4-250 nucleotides long.

The DNA cassette may comprise inverted terminal repeat sequences upstream and downstream of a coding region. The inverted terminal repeat sequences may be symmetrical (i.e. have the same symmetrical three-dimensional organization with respect to each other) or asymmetrical (i.e. have different three-dimensional organization with respect to each other). The inverted terminal repeat sequences may be from the same or different serotypes. An inverted terminal repeat sequence may comprise a terminal resolution site and a Rep binding site.

The DNA cassette (or a cassette sequence) may comprise a homopolymeric sequence. The DNA cassette (or a cassette sequence) may comprise a homopolymeric sequence at a 5'-end or a 3'-end or both a 5'-end and a 3'-end. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides. The homopolymeric sequence may be formed by at least two cassette sequences.

The DNA cassette (or a cassette sequence) may encode a nuclease protein of a CRISPR system (e.g. Cas9, Cpf1 or Mad7), and/or a guide RNA, a DNA polymerase, a reverse transcriptase, or an exonuclease. The DNA cassette (or a cassette sequence) may encode a DNA polymerase, a reverse transcriptase or an exonuclease. The DNA cassette (or a cassette sequence) may encode a nuclease protein of a CRISPR system (e.g. Cas9, Cpf1, or Mad7) and a DNA polymerase, a reverse transcriptase or an exonuclease.

The DNA cassette (or a cassette sequence) may encode a therapeutic target and a T7 or SP6 promoter. The therapeutic target may comprise or consist of functional gene, vaccine antigen or neoantigen. The therapeutic target may be at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 base pairs long.

The DNA cassette (or a cassette sequence) may encode any component of self-amplifying mRNA system. The linear DNA product may comprise (or further comprise) a gene sequence encoding for all the components of a self-amplifying mRNA system. The DNA cassette (or a cassette sequence) may encode tRNA or siRNA.

The DNA cassette (or a cassette sequence) may encode the nuclease of the CRISPR system and/or the guide RNA used to treat any disease or disorder. For example, the linear DNA product encoding the nuclease of the CRISPR system and the linear DNA product encoding the guide RNA may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product encoding the nuclease of the CRISPR system and the linear DNA product encoding the guide RNA is used to treat a monogenic disorder. For example, the linear DNA product encoding the nuclease of the CRISPR system and the linear DNA product encoding the guide RNA, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The DNA cassette (or a cassette sequence) may comprise a coding sequence. The coding sequence may be at least 250, at least 500, at least 750, at least 1000, at least 1500, at least 2000, at least 3000, at least 4000, at least 5000, at least 7500, at least 10,000, at least 15,000, at least 20,000. at least 25,000, or at least 30,000 base pairs (bp) in length. The coding sequence may be formed by at least two cassette sequences. In the embodiments, in which the DNA cassette is single-stranded, the coding sequence may be at least 250, at least 500, at least 750, at least 1000, at least 1500, at least 2000, at least 3000, at least 4000, at least 5000, at least 7500, at least 10,000, at least 15,000, at least 20,000. at least 25,000, or at least 30,000 nucleotides in length

The DNA cassette (or a cassette sequence) may comprise a promoter (or a portion thereof) and a coding sequence. The promoter (or portion thereof) may be operably linked to the coding sequence. The cassette may comprise a promoter, a coding sequence and a 3' untranslated region e.g. a poly(A) tail. The cassette may comprise a promoter, a coding sequence, a 3' untranslated region e.g. a poly(A) tail, and a translational termination sequence. The cassette may comprise a promoter, a ribosome binding site, a coding sequence and a 3' untranslated region e.g. a poly(A) tail. The cassette may comprise a promoter, a ribosome binding site, a coding sequence, a 3' untranslated region e.g. a poly(A) tail,

and a translational termination sequence. The cassette may comprise one or more of: a promoter; a ribosome binding site; a coding sequence; a 3' untranslated region e.g. a poly(A) tail; and a translational termination sequence.

The DNA cassette may comprise a promoter. The promoter may be an inducible or constitutive promoter. The promoter may be, for example, a CMV promoter or a CAG promoter.

The DNA cassette may further comprise an enhancer.

The DNA cassette may comprise an insulator. The insulator may be a cis-regulatory element. The insulator may be at least 300, at least 400, at least 500, at least 1000, at least 1500 or at least 2000 nucleotides in length. The insulator may function either as an enhancer-blocker or a barrier, or both.

The DNA cassette may be an expression cassette. The DNA cassette may be a eukaryotic expression cassette e.g. a mammalian expression cassette or a plant expression cassette. The DNA cassette may be a prokaryotic expression cassette e.g. a bacterial expression cassette.

The DNA cassette may further comprise a reporter gene. The reporter gene may be an eGFP reporter gene or a luciferase reporter gene.

The DNA cassette may additionally comprise a sequence aiding protein expression, e.g. a cap-independent translation element.

The DNA cassette may comprise a gene of interest (GOI). The DNA cassette may comprise the sequence for at least two, three, four, or five genes of interest.

The DNA cassette may comprise (or encode) a repair template (or editing template). The repair template (or editing template) may be for use in CRISPR-Cas mediated homology directed repair (HDR). The DNA cassette may encode a CRISPR guide RNA.

The DNA cassette may further comprise a LoxP sequence, preferably two LoxP sequences. The two LoxP sequences may be oriented in the same direction. In this case, the DNA sequence between the two LoxP sequences may be excised as a circular loop of DNA. The two LoxP sequences may be oriented in the opposite directions. In this case, the DNA sequence between the two LoxP sequences may be inverted. Preferably, the two LoxP sequences are in the same orientation (i.e. the same direction) in the cassette. One LoxP sequence may be at a first end of the cassette and the other LoxP sequence may be at a second end of the cassette. One LoxP sequence may be upstream and the other LoxP sequence may be downstream of the other sequence components of the cassette.

The DNA cassette may be a functional cassette i.e. it may have all sequence elements required for expression of a coding sequence.

Any of the sequence components described above in relation to the DNA cassette may be comprised in a single cassette sequence or split across two or more cassette sequences. For example, a coding sequence may be comprised in a single cassette sequence or split across two or more cassette sequences. A promoter may be comprised in a single cassette sequence or split across two or more cassette sequences.

The first cassette sequence may comprise a promoter and the second cassette sequence may comprise a coding sequence.

The DNA cassette may comprise a modified nucleotide (e.g. an alkynyl modified nucleotide) to enable the appending of an NLS molecule. The DNA cassette may comprise at least 2, at least 3, at least 4, or at least 5 modified nucleotides (e.g. alkynyl modified nucleotides) to enable the appending of at least 2, at least 3, at least 4, or at least 5 NLS molecules.

The single-stranded DNA (ssDNA) cassette may comprise a nuclease-resistant nucleotide at the 5' end of the ssDNA cassette or 5' of the ssDNA cassette, and a nuclease-resistant nucleotides at the 3'-end of the ssDNA cassette or 3' of the ssDNA cassette, wherein the cassette comprises at least 100 nucleotides.

The single-stranded DNA (ssDNA) cassette may comprise at least 3 nuclease-resistant nucleotides at the 5' end of the ssDNA cassette or 5' of the ssDNA cassette, and at least 3 nuclease-resistant nucleotides at the 3'-end of the ssDNA cassette or 3' of the ssDNA cassette, optionally wherein the ssDNA cassette comprises at least 100 nucleotides.

The single-stranded DNA (ssDNA) cassette may comprise at least 5 nuclease-resistant nucleotides at the 5' end of the ssDNA cassette or 5' of the ssDNA cassette, and at least 5 nuclease-resistant nucleotides at the 3'-end of the ssDNA cassette or 3' of the ssDNA cassette, optionally wherein the ssDNA cassette comprises at least 100 nucleotides.

The single-stranded DNA (ssDNA) cassette may comprise x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1, optionally wherein the ssDNA cassette comprises at least 100 nucleotides.

x and y may be independently selected from at least 1, at least 2, at least 3, at least 4 and at least 5. x and y may be different or the same. Preferably x and y are both at least 3. x and y may both be at least 5. x may be 3 and y be 1 and vice versa. x may be 3 and y be 5 and vice versa.

The linear DNA product may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease T and/or exonuclease VII) and/or 5'-end exonuclease digestion (e.g. by exonuclease VIII, exonuclease VII, RecJf, RecJ, T7 exonuclease, Lambda exonuclease and/or T5 exonuclease).

## 2. Methods of production of the linear DNA product

The invention provides a method of producing a linear DNA product as described herein.

The invention also provides a method of producing a linear DNA product, wherein the method comprises:

(a) contacting a double-stranded DNA molecule with an endonuclease, a ligase, a first adaptor molecule, and a second adaptor molecule to form a single contiguous aqueous volume; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the linear DNA product comprises a first adaptor molecule at a first end of the linear double-stranded region and a second adaptor molecule at a second end of the linear double-stranded region; and wherein a nuclear localisation signal (NLS) molecule is appended to the linear DNA product; and

wherein the linear DNA product is resistant to nuclease digestion.

The method may comprise:

(a) contacting a double-stranded DNA molecule with an endonuclease, a ligase, a first adaptor molecule, a second adaptor molecule; and n adaptor molecules to form a single contiguous aqueous volume; and

(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the linear DNA product comprises a first adaptor molecule at a first end of the linear double-stranded region and a second adaptor molecule at a second end of the linear double-stranded region; and wherein a nuclear localisation signal (NLS) molecule is appended to the linear DNA product; and

wherein the linear DNA product is resistant to nuclease digestion.

Each of the n adaptor molecules may (i) separate two sequences (i.e. two cassette sequences) of the double-stranded region; (ii) be between the first adaptor molecule and the first end of the linear double-stranded region; or (iii) be between the second end of the linear double-stranded region and the second adaptor molecule.

The double-stranded DNA molecule may be circular or branched. The double-stranded DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule.

The double-stranded DNA molecule may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise one or more cleavable (e.g. endonuclease) target sequences. The double-stranded DNA molecule may comprise two cleavable (e.g. endonuclease) target sequences. The one or more cleavable (e.g. endonuclease) target sequences may be Type IIS endonuclease target sequences. The one or more cleavable (e.g. endonuclease) target sequences may be Bbsl, Bsal, BsmBI, BspQI, BtgZI, Esp3I,SapI, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMII, BseNI, BseRI, BseXI, Bsgl, BsIFI, BsmAl, BsmFl, Bsml, Bso31I, BspCNI, BspMI, BspPl, BspQI, BspTNI, BsrDI, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, Plel, Ppsl, Psrl, Schl, SfaNl, Taqll, TspDTI and/or TspGWI target sequences.

The double-stranded DNA molecule may be a product of amplification. Preferably, the amplification is rolling circle amplification.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMII, BseNI, BseRI, BseXI, Bsgl, BsIFI, BsmAl, BsmFl, Bsml, Bso31I, BspCNI, BspMI, BspPl, BspQI, BspTNI, BsrDI, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, Plel, Ppsl, Psrl, Schl, SfaNl, Taqll, TspDTI and/or TspGWI restriction enzyme.

The first adaptor molecule and the second adaptor molecule are described in other sections of the application. The NLS molecules are described in other sections of the application.

The step of incubating the single contiguous aqueous volume to generate the linear DNA product may comprise generating the linear portion of the double-stranded DNA molecule by digesting the double-stranded DNA molecule with the endonuclease.

The step of incubating the single contiguous aqueous volume may be performed under conditions that promote appending (or linking) of the first and second adaptor molecules to the linear double-stranded region to produce the linear DNA product. The appending may be performed by creating a covalent link between the first and/or second adaptor molecule and the end(s) of the linear double-stranded region (or linear portion of the double-stranded DNA molecule).

The step of incubating the single contiguous aqueous volume may be performed under conditions that promote digestion of the double-stranded DNA molecule to produce the linear portion of the double-stranded DNA molecule. The digestion of the double-stranded DNA molecule to produce the linear portion of the double-stranded DNA molecule may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear double-stranded region to the first and second adaptor molecules. The ligation may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% efficient. For example, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% of the linear double-stranded regions (or the portions of the double-stranded DNA molecules) may be incorporated into closed linear DNA products. Preferably, the ligation is at least 15% efficient.

Ligation efficiency may be established based on DNA quantification values before and after the digestion/ligation reaction. Thus, ligation efficiency may be established based on the equation:

$$\text{(starting amplified DNA amount)} / \text{(final linear DNA amount)} \times 100\%.$$

Ligation efficiency may also be established based on DNA quantification values before and after the digestion/ligation reaction and the subsequent exonuclease treatment to remove remaining unprotected DNA molecules and adaptor molecules excess.

For example, the amplified DNA products generated by rolling circle amplification is first quantified so that the amount of the amplified DNA products used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear double-stranded region to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is about 37°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous volume does not deviate by more than 5°C, preferably by not more than 3°C, even more preferably not more than 1°C. Preferably, the constant temperature is a temperature in a range of 33°C-40°C.

The method may further comprise, before step (a) (i.e. the step of contacting the double-stranded DNA molecule with the endonuclease, the ligase and the first and second adaptor molecules), a step of amplifying a DNA template molecule to produce the double-stranded DNA molecule. The step of amplifying may be performed by in vitro or in vivo amplification. Preferably, the step of amplifying is performed by in vitro amplification. For example, the step of amplifying may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). The DNA template molecule may be amplified by rolling circle amplification.

Amplification (i.e. the step(s) of amplifying) may be performed using: a primer specific for the DNA template molecule; a pair of primers specific for the DNA template molecule; or performed in the presence of a primase (such as *Thermus thermophilus* (*Tth*) PrimPol (*Tth*PrimPol)). Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Rolling circle amplification may be performed without any primers, or in the presence of a primer or multiple primers. For example, the primer may be a synthetic primer. The primers may be random primers. Rolling circle amplification may be performed in the presence of a primase. The primase may be *Tth*PrimPol. Preferably, if the rolling circle amplification is performed without any primers, it is performed in the presence of a primase, such as *Tth*PrimPol. Similarly, if a primer is used during amplification reaction, a primase is not used. The double-stranded DNA product may be generated by the rolling circle amplification *in vitro* under isothermal conditions using a suitable nucleic acid polymerase, such as Phi29 DNA polymerase.

In the methods described herein, the DNA template molecule may comprise at least one cleavable target sequence. The cleavable target sequence may be an endonuclease target sequence. Thus, the DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a BbsI, BsaI, BsmBI, BspQI, BtgZI, Esp3I,SapI, AarI, Acc36I, AclWI, AcuI, AjuI, AloI, Alw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the linear DNA product.

The DNA template molecule used in the methods described herein may be single-stranded or double-stranded. Preferably, the DNA template molecule is double-stranded. The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from recombinase reaction, preferably Cre recombinase reaction, or (ii) a circular DNA molecule obtained from ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), a DNA template molecule may be circularized to produce a DNA template molecule suitable for use in the methods described herein.

The method may further comprise, after the step of incubating the single contiguous aqueous volume, a step of purification of the linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. This step allows for removal of any double-stranded DNA molecules and/or adaptor molecules which have not been used in the course of performing the method.

The step of incubating the single contiguous aqueous volume (or the purified product) with a nuclease may be performed at a temperature of 5-90°C, 10-80°C, 15-70°C, 20-60°C, 25-50°C, 30-45°C or 35-40°C. The step of incubating the single contiguous aqueous volume (or the purified product) with a nuclease may be performed for at least 10, at least 20, at least 30, at least 40, at least 50, or at least 60 min. The step of incubating the single contiguous aqueous volume (or the purified product) may be performed at two different temperatures. For example, the step of incubating the single contiguous aqueous volume (or the purified product) may be performed at 15-40°C for 10-60 minutes followed by a temperature of 60-90°C for 10-30 min. The higher temperature typically inactivates the nuclease (e.g. exonuclease). Thus, the method further provides a step of inactivating the nuclease (e.g. exonuclease). The step of incubating the single contiguous aqueous volume (or the purified product) may be performed at 37°C for at least 30 min, for example 2 hours. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may further comprise (after the steps of amplification and before the step of forming a single contiguous aqueous volume) a step of heat-deactivation. The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The methods may comprise amplifying DNA template molecules (e.g. first and second DNA template molecules) to generate amplified DNA products (e.g. first and second amplified DNA products), wherein the DNA template molecules comprise at least one cleavable target sequence (e.g. endonuclease target sequence).

Each amplified DNA product may comprise a sequence of the DNA cassette (i.e. a cassette sequence). For example, the first amplified DNA product may comprise a first cassette sequence and the second amplified DNA product may comprise a second cassette sequence.

The amplified DNA product(s) is/are preferably produced by rolling circle amplification. The amplified DNA product(s) may be circular or branched. The amplified DNA product(s) is/are preferably concatemers (e.g. the first amplified DNA product is a first concatemer and the second amplified DNA product is a second concatemer). The concatemers may be generated by rolling circle amplification. The amplified DNA product(s) may each comprise two or more copies of the same cassette sequence. Each amplified DNA product may be a concatemer comprising multiple copies of same cassette sequence. The amplified DNA product(s) may each comprise double-stranded regions and single-stranded regions. Preferably, the amplified DNA product(s) is/are double-stranded DNA molecules. Each amplified DNA product may have features of the double-stranded DNA molecule as described herein.

The method of producing a linear DNA product may comprise the steps of:

(a) amplifying a first DNA template molecule to generate a first amplified DNA product, wherein the first DNA template molecule comprises at least one endonuclease target sequence and wherein the first amplified DNA product is a first concatemer, and amplifying a second DNA template molecule to generate a second amplified DNA product, wherein the second DNA template molecule comprises at least one endonuclease target sequence and wherein the second amplified DNA product is a second concatemer;

(b) forming a single contiguous aqueous volume comprising the first amplified DNA product, the second amplified DNA product, an endonuclease, a ligase, a first adaptor molecule, and a second adaptor molecule; and

(c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product; and wherein the NLS molecule is appended to the linear DNA product.

The method of producing a linear DNA product may comprise the steps of:

(a) amplifying a first DNA template molecule to generate a first amplified DNA product, wherein the first DNA template molecule comprises at least one endonuclease target sequence and wherein the first amplified DNA product is a first concatemer, and amplifying a second DNA template molecule to generate a second amplified DNA product, wherein the second DNA template molecule comprises at least one endonuclease target sequence and wherein the second amplified DNA product is a second concatemer;

(b) forming a single contiguous aqueous volume comprising the first amplified DNA product, the second amplified DNA product, an endonuclease, a ligase, a first adaptor molecule, a second adaptor molecule and a third adaptor molecule; and

(c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product; and wherein the NLS molecule is appended to the linear DNA product.

The method of producing a linear DNA product may comprise the steps of:

(a) amplifying a first DNA template molecule to generate a first amplified DNA product, wherein the first DNA template molecule comprises at least one endonuclease target sequence and wherein the first amplified DNA product is a first concatemer, and amplifying a second DNA template molecule to generate a second amplified DNA product, wherein the second DNA template molecule comprises at least one endonuclease target sequence and wherein the second amplified DNA product is a second concatemer;

(b) forming a single contiguous aqueous volume comprising the first amplified DNA product, the second amplified DNA product, an endonuclease, a ligase, a first adaptor molecule, a second adaptor molecule and n adaptor molecules; and

(c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product; and wherein the NLS molecule is appended to the linear DNA product.

The first adaptor molecule may be at a first end of the linear double-stranded region. The second adaptor molecule may be at a second end of the linear double-stranded region. The third adaptor molecule may (i) separate the linear portion of the first amplified DNA product from the linear portion of the second amplified product; (ii) be between the first adaptor molecule and the first end of the linear double-stranded region; or (iii) be between the second end of the linear double-stranded region and the second adaptor molecule. Each of the n adaptor molecules may (i) separate the linear portion of the first amplified DNA product from the linear portion of the second amplified product; (ii) be between the first adaptor molecule and the first end of the linear double-stranded region; or (iii) be between the second end of the linear double-stranded region and the second adaptor molecule.

In the methods, n adaptor molecules may be 1-100 adaptor molecules.

The methods for producing the linear DNA product comprising a single-stranded DNA cassette are provided in WO2024/017978 A1, which is incorporated herein by reference in its entirety.

**3. Methods for transcription and protein expression**

The invention provides a method for in vitro transcription of a linear DNA product as described herein, wherein the method comprises contacting the linear DNA product with a (RNA) polymerase and producing a transcription product encoded by the linear DNA product.

The invention provides a method for in vitro transcription of a linear DNA product, the method comprising:

(a) providing a linear DNA product as described herein;
(b) contacting the linear DNA product with an RNA polymerase; and
(c) producing a transcription product.

The invention provides a method for producing a protein, wherein the method comprises introducing the linear DNA product as described herein into a cell (e.g. a prokaryotic cell or a eukaryotic cell) or a cell-free expression system to generate a protein encoded by the linear DNA product.

The cell-free expression system may originate from (or be derived from) a prokaryotic cell or eukaryotic cell. For example, the cell-free expression system may originate from (or be derived from) rabbit reticulocytes, wheat germ or Escherichia coli.

The cell may be a prokaryotic cell or a eukaryotic cell. The cell may be an animal cell, such as mammalian cell (e.g. a human cell), a fungal cell, a cell of a micro-organism (e.g. a prokaryotic cell or a eukaryotic cell), or a plant cell. Preferably, the cell is a human cell. The step of introducing the linear DNA product into a cell may be performed in vivo or in vitro.

The linear DNA product may comprise a DNA cassette as described herein. The desired protein might be encoded by the DNA cassette. The step of introducing the linear DNA product into a cell may be performed in vivo or in vitro.

**4. Methods for cell transfection and cell transfection compositions**

The invention provides a method for cell transfection of a linear DNA product as described herein, into a cell.

The invention provides a method for cell transfection of a linear DNA product into a cell, wherein the method comprises:

(b) contacting a cell with the linear DNA product as described herein; and
(c) transfecting the linear DNA product into the cytosol of the cell.

Preferably, the linear DNA product is translocated to the nucleus of the cell.

The invention provides a cell transfection composition comprising a linear DNA product produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

The invention further provides a cell transfection method comprising contacting (in vitro) a cell to be transfected with a linear DNA product as described herein, and wherein the linear DNA product is transfected into the cytosol of the cell.

The cell or cells to be transfected may be provided in a cell culture medium (e.g. in a Petri dish, culture vessel or well, etc). The linear DNA product may be added directly to the cell culture medium or the cells may be added to a solution, such as saline, a buffered solution or a cell culture medium, comprising the linear DNA product.

The carrier may be a viral carrier or a non-viral carrier. Viral carriers include a lentiviral vector, an adenoviral vector, a retroviral vector, or an adeno associate viral vector for delivery of the linear DNA product. Non-viral carriers (or vectors) include complexing the linear DNA product with a cationic agent such as a cationic cell penetrating peptide (CPP);

a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product.

The carrier may be a modification of the linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

The invention further provides a cell obtainable by the methods of the invention. Thus, the cell may comprise a linear DNA product as described herein.

The invention further provides a cell transfected with a linear DNA product as described herein.

The cell may be an animal cell, such as mammalian cell (e.g. a human cell), a fungal cell, a cell of a microorganism (e.g. a prokaryotic cell or a eukaryotic cell), or a plant cell. Preferably, the cell is a human cell.

The step of contacting the cell with a linear DNA product may be performed in vivo or in vitro. For example, the linear DNA product may be administered to an organism (e.g. a subject) in need thereof. The organism (e.g. the subject) may be an animal, such as mammal (e.g. a human), a fungus, a micro-organism, or a plant.

Any or all of the linear DNA products described herein may be delivered to a cell via delivery particles such as liposomes, nanoparticles, exosomes, macrovesicles, viral or non-viral vectors. Any or all of the linear DNA products described herein may be delivered to a cell using a gene-gun. Any or all of the linear DNA products described herein may be delivered to a cell by electroporation. Any or all of the linear DNA products described herein may be delivered to a cell by hydrodynamic needle. The linear DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The linear DNA product and the lipid component may reversibly interact to form a self-assembled nanoparticle. The linear DNA product and the lipid component may reversibly interact in the self-assembled nanoparticle through intermolecular forces. The linear DNA product and the lipid component may reversibly interact in the self-assembled nanoparticle through non-covalent interactions. The linear DNA product and the lipid component may reversibly interact in the self-assembled nanoparticle through hydrogen bonds, van der Waals, hydrophobic, and/or electrostatic interactions. The linear DNA product and the lipid component may not be conjugated or linked by forces other than intermolecular forces in the self-assembled nanoparticle.

## 5. Uses and applications

The linear DNA product as described herein finds application in different fields of biotechnology and pharmacology.

The linear DNA product as described herein is particularly suitable for use in therapy. The invention provides the use of the linear DNA product as described herein in therapy. The linear DNA product may encode a sequence of a therapeutic protein, a part of a vaccine, or an element of a genetic engineering mechanism, which is used to treat a disease or infection in a subject. The invention further provides the linear DNA product as described herein for use as a medicament. The invention also provides the use of a linear DNA product as described herein for the manufacture of a medicament for treating a disease. The invention further provides the linear DNA product as described herein for use in treating a disease.

The linear DNA product described herein may be used to treat any disease or disorder. For example, the linear DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product is used to treat a monogenic disorder. For example, the linear DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The invention provides the use of the linear DNA product as described herein for nuclear expression of a gene of interest.

The invention provides the use of the linear DNA product as described herein for expression of RNA in a cell. The RNA may be miRNA or lncRNA.

The linear DNA product as described herein is very simple in nature (e.g. no bacterial backbone), which means that it is typically easy to work with. The pure and simple nature of the product described herein makes it particularly suitable for use in cell therapy. For example, a cell comprising the linear DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product described herein for use in cell therapy.

Preferably, cell therapy is ex-vivo cell therapy. The cell may be an animal cell, preferably mammal cell, such as human cell.

The invention provides the use of a linear DNA product described herein in the production of a CAR-T cell.

The invention provides a method for producing a genetically engineered CAR-T cell comprising: (a) introducing the linear DNA product described herein into a T cell; and (b) expressing a gene of interest encoded by the linear DNA product. Preferably, the gene of interest is a tumour-specific CAR.

The method may further comprise, before step (a) (i.e. introducing the linear DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

The invention also provides the use of the linear DNA product as described herein in gene editing.

The invention also provides the use of the linear DNA product as described herein in gene therapy.

The linear DNA product described herein is particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product may comprise a gene sequence encoding any component of the CRISPR machinery. The linear DNA product may encode all components of the CRISPR machinery.

The linear DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000. 15000, or 20000 base pairs long. The linear DNA product encoding the repair template may be delivered to a cell by a nanoparticle, a non-viral vector or a viral vector, or without the aid of any carrier. The linear DNA product encoding the repair template may be delivered to a cell by electroporation. The linear DNA product encoding the repair template may be delivered to a cell by hydrodynamic needle.

The linear DNA product may comprise (or further comprise) a gene sequence encoding a nuclease protein of a CRISPR system (e.g. Cas9, Cpf1 or Mad7), and/or a guide RNA. The linear DNA product may comprise (or further comprise) a gene sequence encoding a nuclease protein of a CRISPR system (e.g. Cas9, Cpf1, or Mad7). The linear DNA product may comprise (or further comprise) a gene sequence encoding a guide RNA. The linear DNA product may comprise (or further comprise) a gene sequence encoding a nuclease protein of a CRISPR system (e.g. Cas9, Cpf1, or Mad7) and a guide RNA. The linear DNA product may comprise (or further comprise) a gene sequence encoding a genomic target to be modified (e.g. a spacer). The linear DNA product may be ligated to a vector. The vector may comprise a sequence of some of the components of the CRISPR system. The vector may comprise a sequence of guide RNA or a sequence of a part of the guide RNA. If the vector comprises a sequence of a part of the guide RNA, the linear DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product may encode the nuclease of the CRISPR system and guide RNA. One linear DNA product may encode the nuclease of the CRISPR system, and the other linear DNA product may encode guide RNA.

The linear DNA product may comprise (or further comprise) a gene sequence encoding a nuclease protein of a CRISPR system (e.g. Cas9, Cpf1 or Mad7), and/or a gene sequence encoding a DNA polymerase, a reverse transcriptase, or an exonuclease. The linear DNA product may comprise (or further comprise) a gene sequence encoding a DNA polymerase, a reverse transcriptase or an exonuclease. The linear DNA product may comprise (or further comprise) a gene sequence encoding a nuclease protein of a CRISPR system (e.g. Cas9, Cpf1, or Mad7) and a gene sequence encoding a DNA polymerase, a reverse transcriptase or an exonuclease.

The linear DNA product may be for use in CRISPR-Cas mediated repair by recombination, homology-directed repair, or non-homologous end joining.

If the nuclease of the CRISPR system and the guide RNA are encoded by a different linear DNA product, they may be part of a different or the same delivery mechanism. For example, the linear DNA product encoding the nuclease of the CRISPR system may be delivered to a cell by a first nanoparticle, non-viral vector or viral vector, whereas the linear DNA product encoding the guide RNA may be delivered to a cell by a second nanoparticle, a non-viral vector, or viral vector. For example, the linear DNA product encoding the nuclease of the CRISPR system and the linear DNA product encoding the guide RNA may be delivered to a cell by the same nanoparticle, non-viral vector or viral vector.

If the nuclease of the CRISPR system and the guide RNA are encoded by the same linear DNA product (or by a vector that comprises the linear DNA product) they may be part of the same delivery mechanism. For example, the linear DNA product, or the vector, encoding the nuclease of the CRISPR system and the guide RNA may be delivered to a cell by a nanoparticle, a non-viral vector or a viral vector.

The linear DNA product encoding the nuclease of the CRISPR system and the linear DNA product encoding the guide RNA may be delivered to a cell by electroporation. The linear DNA product encoding the nuclease of the CRISPR system and the linear DNA product encoding the guide RNA may be delivered to a cell by hydrodynamic needle.

Thus, the invention provides the linear DNA product described herein for use in CRISPR system delivery to a cell. The invention provides a method of delivering the CRISPR system to a cell, comprising contacting the linear DNA product described herein with a cell.

The cell may be an animal cell, preferably mammal cell, such as human cell.

The invention also provides a cell obtainable by the methods described herein. The cell is particularly suitable for use in cell therapy and/or in vivo therapy.

The linear DNA product as described herein may be used in transcription, to generate RNA, preferably mRNA, in vitro or in vivo.

The linear DNA product may comprise (or further comprise) a sequence encoding for self-amplifying mRNA (SAM). The linear DNA product may comprise (or further comprise) a sequence encoding for tRNA. The linear DNA product may comprise (or further comprise) a sequence encoding for circular mRNA (or circRNA).

The invention provides a use of a linear DNA product as described herein in the production of a viral or non-viral delivery system.

The invention provides a use of a linear DNA product in the production of a viral or non-viral delivery system.

The invention provides a viral or non-viral delivery system comprising a linear DNA product as described herein.

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli*; 3) the incorporation of the plasmid backbone into the viral capsid (e.g. AAV capsid) might be challenging (e.g. issues with antibiotic resistance markers). Methods of producing viral vectors may be performed using other cell lines. For example, a cell line suitable for production of viral vectors may be a Vero cell, or any other stable cell line. Together these represent the main bottleneck in viral vector production (e.g. AAV production). Thus, the linear DNA product described herein is suitable for use in production of viral vectors. The linear DNA product overcomes the above issues with plasmid vectors.

The invention provides a method of producing a viral vector, the method comprising introducing the linear DNA product as described herein into a cell under conditions such that the viral vector is produced. The linear DNA product may encode at least one element required for the production of the viral vector. For example, the linear DNA product may encode Rep and/or Cap elements. The linear DNA product may encode the helper plasmid elements. The linear DNA product may encode Rep, Cap and helper plasmid elements. The linear DNA product may encode a transgene. The method may be an in vivo or in vitro method. The cell may be an animal cell, preferably mammalian cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the linear DNA product) to a cell, comprising contacting the viral vector produced by the methods described herein with the cell. The cell may be an animal cell, preferably mammalian cell, such as human cell.

The invention also provides a cell obtainable by the methods described herein.

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product as described herein is suitable for use in production of non-viral vectors. The linear DNA product produced or obtainable by the methods of the invention overcomes the issues of using plasmid vectors in non-viral vector preparation. For example, the linear DNA product, unlike plasmid DNA, does not comprise a bacterial backbone, allowing more transgene copies per mg of DNA. In addition, the linear DNA product does not comprise antibiotic resistant genes and bacterial contaminants. Moreover, the linear DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product with the cell. The cell may be an animal cell, preferably mammalian cell, such as human cell.

The invention also provides a cell obtainable by the methods described herein.

The linear DNA product as described herein is particularly suitable for use in vaccine production. A vaccine may comprise a linear DNA product described herein. Alternatively, the linear DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2). The linear DNA product may be used to produce a seasonal, pandemic, prophylactic or therapeutic vaccine. The linear DNA product as described herein is particularly suitable for use in personalised vaccine production.

Thus, the invention provides the use of the linear DNA product described herein in the production of a vaccine.

The linear DNA product may encode an antigen, which may cause an immune response in a subject. The subject may be human. Preferably, the antigen is encoded on the DNA cassette. The linear DNA product may encode a neoantigen, which may cause an immune response in a subject. Preferably, the neoantigen is encoded on the DNA cassette.

## 6. Kits

The invention provides a kit comprising components required to carry out the methods described herein.

The kit may comprise:

(a) a strand displacing polymerase;
(b) an endonuclease;
(c) a ligase; and
(d) optionally first and second adaptor molecules.

The strand-displacing polymerase may be Phi29 DNA polymerase or a mutant thereof which retains functionality (e.g. QualiPhi®, a chimeric form of Phi29 DNA polymerase from 4basebio).

The kit may further comprise an exonuclease e.g. exonuclease I, exonuclease III and/or exonuclease VIII.

The endonuclease may be any endonuclease described herein. Preferably, the endonuclease is a Type IIS restriction enzyme.

The ligase may a DNA ligase, such as a T4 DNA ligase, T7 DNA ligase, mammalian DNA ligase I, III and IV; Taq DNA ligase, Tth DNA ligase, or E. coli DNA ligase.

The foregoing description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

## BRIEF DESCRIPTION OF THE FIGURES

Figure 1 illustrates an example of a NLS molecule conjugated to a DNA product with different types of adaptors.

Figure 2 illustrates examples of sequences of the adaptor molecules conjugated to NLS peptide(s). The adaptor comprising SEQ ID NOs: 25 & 26, conjugated with an NLS peptide (SEQ ID NO: 4) at the 3' end of the top strand. The bottom strand comprising 5 phosphate at the 5' end essential to the ligation. Both strands include phosphorothioate (PS) modifications at the free blunt ends (5' & 3'), which make the final product resistant to exonucleases. This adaptor can be used to produce a linear DNA product with protected nucleotides in each strand.

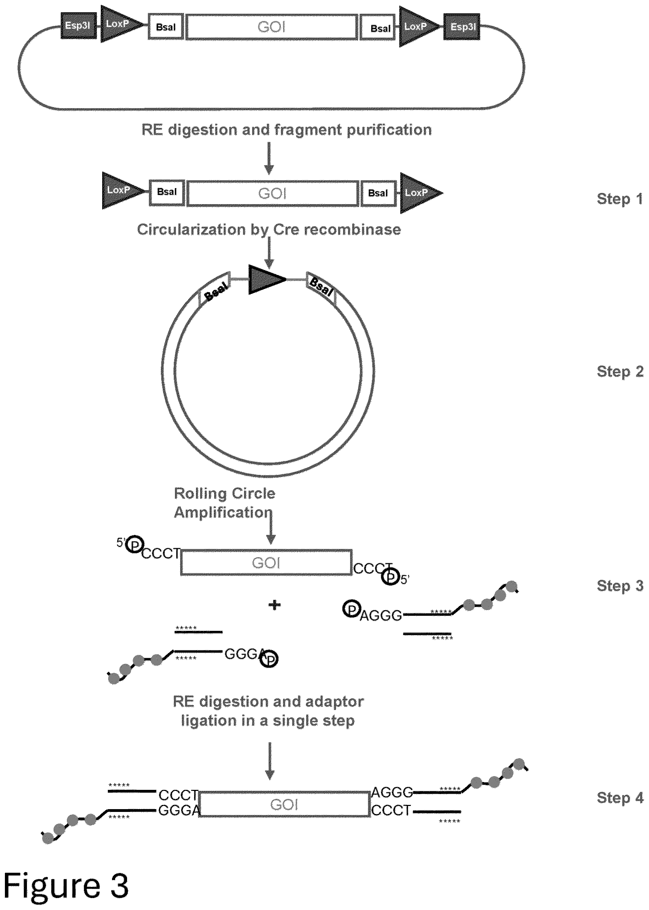

Figure 3 illustrates the workflow to obtain the DL product (a sequence ligated in one single reaction with adaptors on both ends that make the final product resistant to exonucleases). Sequence was digested using restriction enzyme (step 1) and loxp present on either side of the sequences were recombined using CRE recombinase (step 2) to form circular product required for rolling circle amplification to amplify the sequences (step 3). In step 4, restriction digestion and ligation happen simultaneously where the sequence that was amplified separately is put together in one reaction along with custom adaptors conjugated with the NLS peptide (Peptide-oligo-conjugate) to form single construct.

Figure 4 shows agarose gel (0.8%) image showing the DL product obtained after digestion ligation, T7 endonuclease and exonucleases treatments. Green boxes highlight the peptide oligo-conjugated DNA product.

Figure 5 shows agarose gel (0.8%) image showing the DL product obtained after digestion ligation using different ligases and exonucleases treatments.

Figure 6 shows agarose gel (0.8%) image showing the DL product obtained after digestion ligation, T7 endonuclease and exonucleases treatments. Green boxes highlight the peptide oligo-conjugated DNA product.

Figure 7 shows agarose gel (0.8%) image showing the DL product obtained after digestion ligation and exonucleases treatments. A. Green boxes highlight the peptide oligo-conjugated DNA product. Blue Box highlights the DNA product containing the RNA Aptamer. B. DNA products after extra exonucleases and T7 endonuclease treatment.

Figure 8 Illustrates the workflow to obtain a DNA product comprising an NLS molecule which is an RNA Aptamer.

Figure 9 shows agarose gel (0.8%) image showing the DL product obtained after digestion ligation and exonucleases treatments.

Figure 10 illustrates examples of suitable conjugation methods for appending the NLS molecule to the linear DNA product.

## EXAMPLES

### Example 1 - Generation of NLS peptide - DNA construct

A DNA construct sized ~2200bp (which included a gene of interest encoding GFP), containing a CMV promoter was amplified using rolling circle amplification (RCA) with QualiPhi® (a chimeric form of Phi29 DNA polymerase, 4basebio) Qualiph polymerase. Priming of the template DNA strands was performed using Tth PrimPol enzyme, that primes DNA in sequence-independent fashion, or with a pair of sequence-specific primers. The reaction was incubated under the following RCA conditions:
Before amplification, circularized DNA template was first denatured by adding 1 volume of buffer D (400 mM KOH, 10 mM EDTA) and incubated for 3 min at room temperature. Samples were then neutralized by adding 1 volume of buffer N (400 mM HCl, 600 mM Tris-HCl pH 7.5). Rolling circle amplification was performed in 1 ml reaction volume, 100 $\mu$l TruePrime WGA reaction buffer 10x (4basebio), 50 $\mu$l denatured DNA sample, 4.6$\mu$lTthPrimPol (21.8 $\mu$M), 16 $\mu$l QualiPhi Phi29 DNA polymerase (12,5 $\mu$M), 2.5 $\mu$l Pyrophosphatase (2.47 $\mu$M) and 40 $\mu$l dNTPs (25 mM). Incubation time and temperature: 20 hours at 30°C, followed by 10 min at 65°C.
To produce the DNA conjugated to an NLS peptide, the DNA generated by Rolling circle amplification (RCA) is digested then ligated to the NLS peptide oligo conjugated adaptors in a single-step, single-volume reaction. This ligation approach allows us to introduce the specific NLS feature in the DNA constructs. The sequences of the adaptors were:

5' GGCCAACAAATGTTAG 3' (SEQ ID NO: 25)
3' CCGGTTGTTTACAATCGGGA/5'phos/ (SEQ ID NO: 26)

Digestion of these sequences produces 5' overhang on either side; upstream and downstream 5' overhang of gene of interest (GOI) (here GOI is GFP) is complementary to the adaptor overhangs.
The DNA product can be obtained by ligating together the digested RCA monomers with the adaptors on both ends to protect the DNA from exonucleases activity and covalently link the NLS peptide.
In the following experiments, the GOI and the adaptors can be ligated in single reaction using different ligases. The digestion/ligation (DL) reaction was conducted for 18hrs. T7 ligase was used to generate the final product at an incubation temperature of 37°C. Treatment with the exonucleases was performed to remove non-ligated DNA. T4 ligase or other ligases could be used to produce the same product. Similarly, different incubation temperatures and time can be used.

To generate the final DNA product, amplified DNAs were incubated with Type II restriction enzyme BsaI, T7 DNA ligase and complementary adaptors to the 5' protruding ends generated by BsaI on the amplified DNA. Digestion and ligation reaction was performed in a single reaction volume of 1mL, containing reaction buffer for DNA ligase 10x (500 mM Tris HCl pH 7.5, 100 mM MgCl2, 100 mM DTT), RCA material (240ng DNA/uL), adaptors (10x of molar excess), T7 Ligase (1.6 ng/uL or 8.6ng/uL, 4basebio), BsaI (6 ng/uL, 4Basebio). The reaction was incubated for 20 hours at 37°C.

Sample is treated with Exonuclease I and III (6.6 ng/uL and 12.6ng/uL respectively) to remove any non-ligated DNA. The reaction was incubated at 37°C for 2hr.

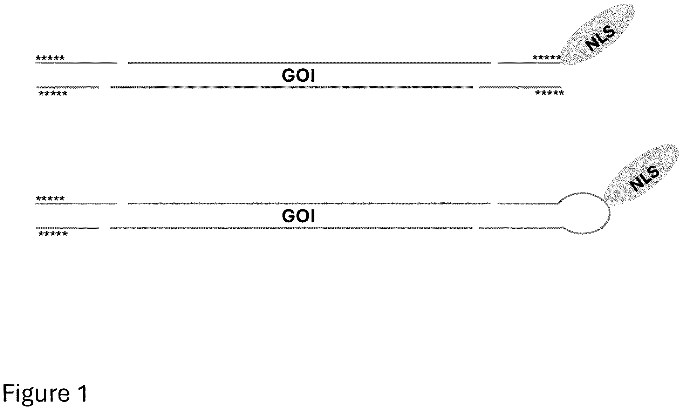

Figure 1 illustrates an example of an NLS molecule conjugated to a DNA product with different types of adaptors.

Figure 2 illustrates examples of sequences of the adaptor molecules conjugated to NLS peptide(s). The adaptor comprising SEQ ID NOs: 25 and 26, conjugated with an NLS peptide at the 3' end of the top strand. The bottom strand comprising 5 phosphate at the 5' end essential to the ligation. Both strands include phosphorothioate (PS) modifications at the free blunt ends (5' & 3'), which make the final product resistant to exonucleases. This adaptor can be used to produce a linear DNA product with protected nucleotides in each strand.

Figure 3 illustrates the workflow to obtain the DL product (a sequence ligated in one single reaction with adaptors on both ends that make the final product resistant to exonucleases). Sequence was digested using restriction enzyme (step 1) and loxp present on either side of the sequences were recombined using CRE recombinase (step 2) to form circular product required for rolling circle amplification to amplify the sequences (step 3). In step 4, restriction digestion and ligation happen simultaneously where the sequence that was amplified separately is put together in one reaction along with custom adaptors conjugated with the NLS peptide (Peptide-oligo-conjugate) to form single construct.

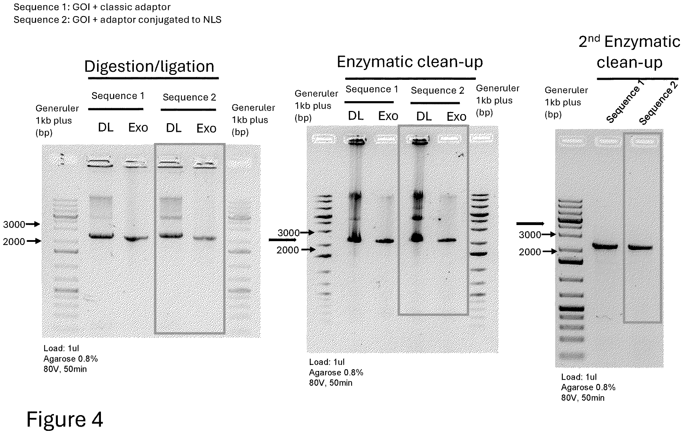

Figure 4 shows agarose gel (0.8%) image showing the DL product obtained after digestion ligation, T7 endonuclease and exonucleases treatments. Green boxes highlight the peptide oligo-conjugated DNA product.

Table 4. The DNA concentration measurement using Qubit for DL samples treated with exonuclease and further exonuclease treatment.

| | Ligases | After DL (ng/ul) | After exonuclease treatment (ng/ul) | After Enzymatic clean-up (ng/ul) | After 2nd Enzymatic clean-up (ng/ul) |
|---|---|---|---|---|---|
| Sequence 1 | T7 | 640; 630 | 302; 320 | 120; 129 | 79.2 |
| Sequence 2 | T7 | 600; 630 | 298; 242 | 86.3; 81.6 | 52.4 |

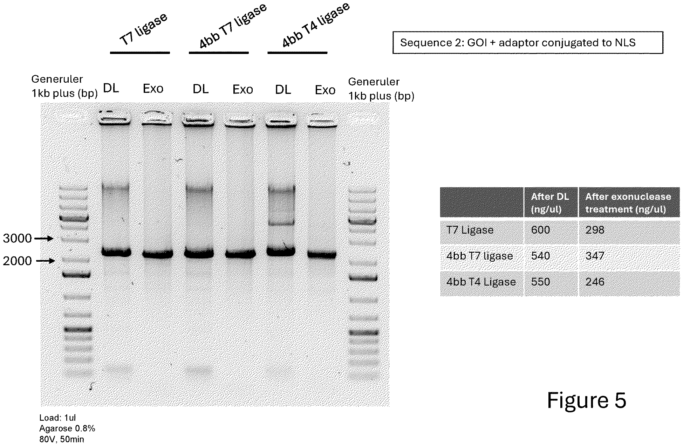

Figure 5 shows agarose gel (0.8%) image showing the DL product obtained after digestion ligation using different ligases and exonucleases treatments. The table shows the DNA concentration measurement using Qubit for DL samples treated with exonuclease and further exonuclease treatment.

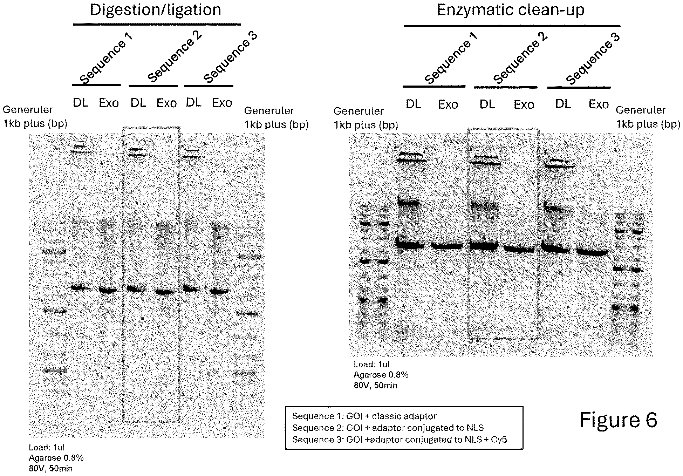

Figure 6 shows agarose gel (0.8%) image showing the DL product obtained after digestion ligation, T7 endonuclease and exonucleases treatments. Green boxes highlight the peptide oligo-conjugated DNA product.

Table 5. The DNA concentration measurement using Qubit for DL samples treated with exonuclease and further exonuclease treatment. Sequence 1: GOI + classic adaptor; Sequence 2: GOI + adaptor conjugated to NLS; and Sequence 3: GOI +adaptor conjugated to NLS + Cy5.

| | Ligases | After DL (ng/ul) | After exonuclease treatment (ng/ul) | After Enzymatic clean-up (ng/ul) |
|---|---|---|---|---|
| Sequence 1 | T7 | 315 | 125; 112 | 82.4; 119 |
| Sequence 2 | T7 | 283 | 108; 134 | 75; 89.4 |
| Sequence 3 | T7 | 295 | 127; 119 | 87.6; 96.9 |

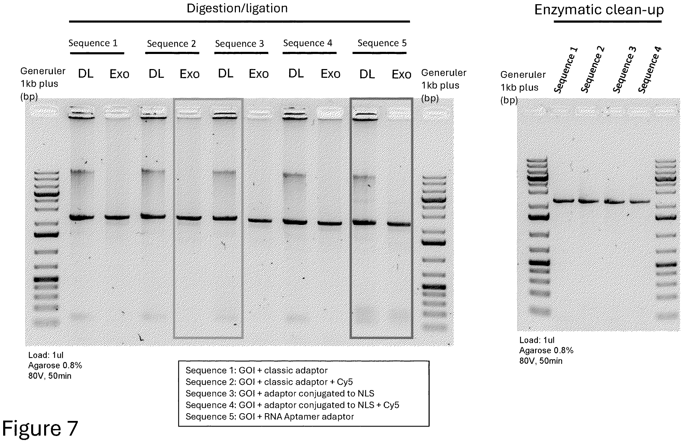

Figure 7 shows (left) agarose gel (0.8%) image showing the DL product obtained after digestion ligation and exonucleases treatments. Green boxes highlight the peptide oligo-conjugated DNA product. Blue Box highlights the DNA product containing the RNA Aptamer. (right) DNA products after extra exonucleases and T7 endonuclease treatment.

Table 6. The DNA concentration measurement using Qubit for DL samples treated with exonuclease and further exonuclease treatment. Sequence 1: GOI + classic adaptor; Sequence 2: GOI + classic adaptor + Cy5; Sequence 3: GOI + adaptor conjugated to NLS; Sequence 4: GOI + adaptor conjugated to NLS + Cy5; and Sequence 5: GOI + RNA Aptamer adaptor.

| | Ligases | After DL (ng/ul) | After exonuclease treatment (ng/ul) | After Enzymatic clean-up (ng/ul) |
|---|---|---|---|---|
| **Sequence 1** | T7 | 242 | 115; 117 | 7.98 |
| **Sequence 2** | T7 | 229 | 115; 132 | 8.1 |
| **Sequence 3** | T7 | 219 | 93.3; 98.7 | 6.9 |
| **Sequence 4** | T7 | 178 | 85.3; 94 | 4.2 |
| **Sequence 5** | T7 | 208 | 64.8; 65.3 | - |

## Example 2 - Generation of NLS aptamer - DNA construct

DNA construct sized ~2200bp, was amplified using rolling circle amplification (RCA) with Qualiph polymerase. Reaction was incubated at 30°C for 20 hours under standard RCA conditions as in Example 1. RCA reaction was then digested and ligated in single reaction using BsaI and T7 ligase and adaptors comprising an RNA aptamer of the sequence: /5Phos/AG GGC TAA CAT TTG rGrArU rCrCrU rGrArG rCrUrA rCrUrG rArCrU rUrUrG rGrGrU rCrGrA rArUrU rGrGrU rUrGrG rCrUrC rGrCrC rCrUrC rUrUrC rUrUrG rGrArA rUrUrC rArGrG rArGrG rGrCrG rArUrU rGrArA rCrGrG rArCrA rCrCrA rCrUrA rCrUrG rArCrC rArUrA rCrArC CAA ATG TTA G (SEQ ID NO: 27). The "r" in the sequence denotes a ribose-based nucleotide (i.e. the RNA part of the RNA/DNA hybrid).

Thus, during the DL step, adaptors which comprise a segment of DNA sequence containing the overhang needed for the ligation and a segment of RNA comprising the Aptamer sequence and forming a hairpin have been used.

Figure 8 Illustrates the workflow to obtain DNA product comprising RNA Aptamer.

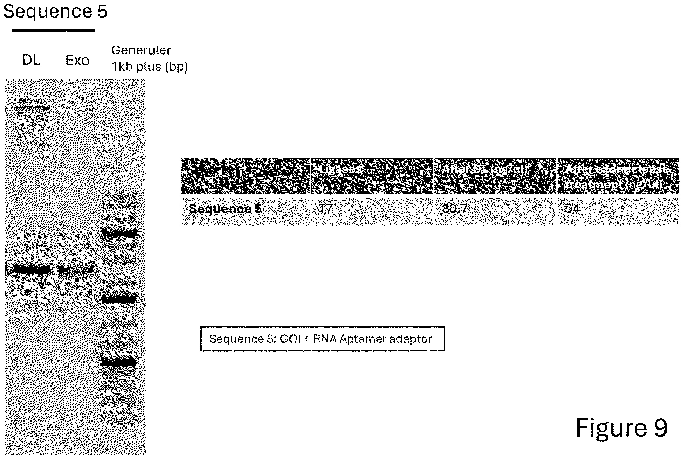

Figure 9 shows agarose gel (0.8%) image showing the DL product obtained after digestion ligation and exonucleases treatments. The table shows the DNA concentration measurement using Qubit for DL samples treated with exonuclease and further exonuclease treatment.

## Claims

1. A linear deoxyribonucleic acid (DNA) product comprising:

   a) a linear double-stranded region;
   b) a first adaptor molecule at a first end of the linear double-stranded region; and
   c) a second adaptor molecule at a second end of the linear double-stranded region;

   wherein the linear DNA product is resistant to exonuclease digestion; and wherein a nuclear localisation signal (NLS) molecule is appended to the linear DNA product.

2. The linear DNA product of claim 1, wherein the NLS molecule is appended to the first adaptor molecule, the second adaptor molecule, or a third adaptor molecule comprised in the linear double-stranded region.

3. The linear DNA product of claim 1 or claim 2, wherein the first adaptor molecule and/or the second adaptor molecule comprise at least 1, at least 2, at least 3, at least 4, or at least 5 phosphorothioated nucleotides.

4. The linear DNA product of any one of claims 1-3, wherein the first adaptor molecule and/or the second adaptor molecule comprise a double stranded portion, optionally wherein the first adaptor molecule and/or the second adaptor molecule comprise at least 1, at least 2, at least 3, at least 4, or at least 5 phosphorothioated nucleotides in each strand of the double stranded portion.

5. The linear DNA product of any one of claims 1-4, wherein at least two NLS molecules are appended to the linear DNA product, optionally wherein the first of the at least two NLS molecules is appended to the first adaptor molecule and the second of the at least two NLS molecules is appended to the second adaptor molecule.

6. The linear DNA product of any one of claims 1-5, wherein the linear DNA product is resistant to 3'- and/or 5'-exonuclease digestion, preferably 3'- and 5'- exonuclease digestion.

7. The linear DNA product of any one of claims 1-6, wherein the NLS molecule is appended to the linear DNA product by a linker, optionally wherein the linker is 4-(N-maleimidomethyl)-cyclohexane-1-carboxylic acid N-hydroxysuccinimide ester (SMCC), or SPDP NHS ester.

8. The linear DNA product of any one of claims 1-7, wherein the linear double stranded region comprises a third adaptor molecule, optionally wherein the third adaptor molecule (i) separates two different sequences of the linear double-stranded region; (ii) is between the first adaptor molecule and the first end of the linear double-stranded region; or (iii) is between the second end of the linear double-stranded region and the second adaptor molecule.

9. The linear DNA product of any one of claims 1-8, wherein the NLS molecule is a NLS peptide, a DNA molecule, or an RNA aptamer.

10. The linear DNA product of claim 9, wherein the NLS peptide comprises a sequence of either one of SEQ ID NOs: 1-24, 27 or 28.

11. A method of producing a linear DNA product, wherein the method comprises:

(a) contacting a double-stranded DNA molecule with an endonuclease, a ligase, a first adaptor molecule, and a second adaptor molecule to form a single contiguous aqueous volume; and
(b) incubating the single contiguous aqueous volume to generate the linear DNA product, wherein the linear DNA product comprises a linear double-stranded region, wherein the linear double-stranded region comprises a linear portion of the double-stranded DNA molecule, and wherein the linear DNA product comprises a first adaptor molecule at a first end of the linear double-stranded region and a second adaptor molecule at a second end of the linear double-stranded region; and wherein a nuclear localisation signal (NLS) molecule is appended to the linear DNA product; and

wherein the linear DNA product is resistant to exonuclease digestion.

12. The method of claim 11, wherein the method further comprises a step of amplifying a DNA template molecule comprising at least one endonuclease target sequence to generate a double-stranded DNA molecule, optionally wherein the DNA template molecule is amplified by rolling circle amplification.

13. The method of claim 11, or claim 12, wherein the method comprises the steps of:

(a) amplifying a first DNA template molecule to generate a first amplified DNA product, wherein the first DNA template molecule comprises at least one endonuclease target sequence and wherein the first amplified DNA product is a first concatemer, and amplifying a second DNA template molecule to generate a second amplified DNA product, wherein the second DNA template molecule comprises at least one endonuclease target sequence and wherein the second amplified DNA product is a second concatemer;
(b) forming a single contiguous aqueous volume comprising the first amplified DNA product, the second amplified DNA product, an endonuclease, a ligase, a first adaptor molecule, a second adaptor molecule and a third adaptor molecule; and
(c) incubating the single contiguous aqueous volume to generate the linear DNA product, wherein the linear DNA product comprises a linear double-stranded region, wherein the linear double-stranded region comprises a linear portion of the first amplified DNA product and a linear portion of the second amplified DNA product; and wherein the NLS molecule is appended to the linear DNA product.

14. The method of any one of claims 11-13, wherein the NLS molecule is appended to the first adaptor molecule, the second adaptor molecule and/or the third adaptor molecule.

15. Use of the linear DNA product of any one of claims 1-10 for nuclear expression of a gene of interest.

Figure 1

Adaptor comprising the POC (Peptide Oligo
Conjugate)

Sticky ends

5′ (P) AGGGCTAACATTTGT*T*G*G*C*C ~~CGKRKLITSEEERSPAKRGRKS
3′      GATTGTAAACA*A*C*C*G*G

Free blunt ends

Sticky ends

5′ (P) AGGGCTAACATTTGT*T*G*G*C*C ~~CGKRKLITSEEERSPAKRGRKS
3′      GATTGTAAACA*A*C*C*G*G ~~CGKRKLITSEEERSPAKRGRKS

(P)   5' Phosphate

* The phosphorothioate (PS) modifications

# Figure 2

Figure 3

Sequence 1: GOI + classic adaptor
Sequence 2: GOI + adaptor conjugated to NLS

Figure 4

Figure 5

Load: 1ul
Agarose 0.8%
80V, 50min

|  | After DL (ng/ul) | After exonuclease treatment (ng/ul) |
|---|---|---|
| T7 Ligase | 600 | 298 |
| 4bb T7 ligase | 540 | 347 |
| 4bb T4 Ligase | 550 | 246 |

Figure 6

Digestion/ligation

Enzymatic clean-up

Sequence 1   Sequence 2   Sequence 3   Sequence 4   Sequence 5

Generuler
1kb plus
(bp)

DL   Exo   DL   Exo   DL   Exo   DL   Exo   DL   Exo

Generuler
1kb plus
(bp)

Generuler
1kb plus
(bp)

Sequence 1   Sequence 2   Sequence 3   Sequence 4

Load: 1ul
Agarose 0.8%
80V, 50min

Sequence 1: GOI + classic adaptor
Sequence 2: GOI + classic adaptor + Cy5
Sequence 3: GOI + adaptor conjugated to NLS
Sequence 4: GOI + adaptor conjugated to NLS + Cy5
Sequence 5: GOI + RNA Aptamer adaptor

Load: 1ul
Agarose 0.8%
80V, 50min

Figure 7

40

Figure 8

# Sequence 5

DL     Exo     Generuler
1kb plus (bp)

| | Ligases | After DL (ng/ul) | After exonuclease treatment (ng/ul) |
|---|---|---|---|
| Sequence 5 | T7 | 80.7 | 54 |

Sequence 5: GOI + RNA Aptamer adaptor

Figure 9

# Oligo peptide conjugate

## Conjugation method 1:

Oligo with amino group    SMCC                       Peptide             Peptide Oligo Conjugation

## Conjugation method 2:

Oligo with amino group    SPDP                       Peptide             Peptide Oligo Conjugation

## Conjugation method 3:

Oligo(Alkynyl modifed)     Peptide                        Peptide Oligo Conjugation

# Figure 10

Europäisches
Patentamt

European
Patent Office

Office européen
des brevets

# EUROPEAN SEARCH REPORT

Application Number

EP 24 19 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JOHANSSON P ET AL: "PCR-generated linear DNA fragments utilized as a hantavirus DNA vaccine", VACCINE, ELSEVIER, AMSTERDAM, NL, vol. 20, no. 27-28, 10 September 2002 (2002-09-10), pages 3379-3388, XP004378533, ISSN: 0264-410X, DOI: 10.1016/S0264-410X(02)00265-7 | 1-10,15 | INV. C12N15/87 C12Q1/6806 |
| Y | * page 3380, right-hand column, paragraph 2 - paragraph 3; figure 1B * * page 3381, left-hand column, paragraph 2; figure 2 * * figure 3 * ----- | 11-14 | |
| X | AA VAN DER M A E M ET AL: "An NLS peptide covalently linked to linear DNA does not enhance transfection efficiency of cationic polymer based gene delivery systems", THE JOURNAL OF GENE MEDICINE, JOHN WILEY & SONS, INC, US, vol. 7, no. 2, 1 February 2005 (2005-02-01), pages 208-217, XP002471232, ISSN: 1099-498X, DOI: 10.1002/JGM.643 | 1-10,15 | |
| | | | **TECHNICAL FIELDS SEARCHED (IPC)** |
| | | | C12N C12Q |
| Y | * page 209, right-hand column - page 210, left-hand column; figures 1, 3 * ----- | 11-14 | |
| X | WO 2023/006978 A1 (4BASEBIO S L U [ES]; 4BASEBIO UK LTD [GB]) 2 February 2023 (2023-02-02) | 1-10 | |
| Y | * page 19, line 1 - page 20, line 4 * ----- | 11-14 | |
| Y | WO 2019/204664 A2 (APDN BVI INC; HUGHES STEPHEN [US] ET AL.) 24 October 2019 (2019-10-24) * claim 7 * ----- | 11-14 | |

-/--

The present search report has been drawn up for all claims

| Place of search | Date of completion of the search | Examiner |
|---|---|---|
| The Hague | 9 January 2025 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................
& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 19 2194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZUBER G ET AL: "Towards synthetic viruses", ADVANCED DRUG DELIVERY REVIEWS, vol. 52, no. 3, 19 November 2001 (2001-11-19), pages 245-253, XP093237836, Amsterdam , NL ISSN: 0169-409X, DOI: 10.1016/S0169-409X(01)00213-7 Retrieved from the Internet: URL:https://www.sciencedirect.com/science/article/pii/S0169409X01002137?via%3Dihub> * figure 7 * | 1,5 | |
| A | SEDELNIKOVA O A ET AL: "Sequence-specific gene cleavage in intact mammalian cells by 125I-labeled triplex-forming oligonucleotides conjugated with nuclear localization signal peptide", ANTISENSE & NUCLEIC ACID DRUG DEVELOPMENT, MARY ANN LIEBERT, INC., NEW YORK, US, vol. 12, no. 1, 1 February 2002 (2002-02-01), pages 43-49, XP002490180, ISSN: 1087-2906, DOI: 10.1089/108729002753670256 * figure 1 * | 1,7 | |
| | | | TECHNICAL FIELDS SEARCHED (IPC) |
| A | JP 2017 086027 A (NAT INST ADVANCED IND SCIENCE & TECH) 25 May 2017 (2017-05-25) * the whole document * | 9,10 | |

The present search report has been drawn up for all claims

| Place of search | Date of completion of the search | Examiner |
|---|---|---|
| The Hague | 9 January 2025 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                 

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 24 19 2194

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2025

| Patent document cited in search report | Publication date | Patent family member(s) | Publication date |
|---|---|---|---|
| WO 2023006978 A1 | 02-02-2023 | AU 2022320939 A1 | 29-02-2024 |
| | | CA 3224561 A1 | 02-02-2023 |
| | | CN 117980501 A | 03-05-2024 |
| | | EP 4124660 A1 | 01-02-2023 |
| | | ES 2947031 T6 | 02-08-2023 |
| | | ES 2956243 A2 | 15-12-2023 |
| | | GB 2621500 A | 14-02-2024 |
| | | GB 2631830 A | 15-01-2025 |
| | | JP 2024528060 A | 26-07-2024 |
| | | KR 20240038989 A | 26-03-2024 |
| | | US 2024076659 A1 | 07-03-2024 |
| | | WO 2023006978 A1 | 02-02-2023 |
| WO 2019204664 A2 | 24-10-2019 | US 2019247437 A1 | 15-08-2019 |
| | | US 2021353676 A1 | 18-11-2021 |
| | | WO 2019204664 A2 | 24-10-2019 |
| JP 2017086027 A | 25-05-2017 | JP 6619211 B2 | 11-12-2019 |
| | | JP 2017086027 A | 25-05-2017 |

EPO FORM P0459

For more details about this annex : see Official Journal of the European Patent Office, No. 12/82

## REFERENCES CITED IN THE DESCRIPTION

**Patent documents cited in the description**

- WO 2023006978 A1

- WO 2024017978 A1

**Non-patent literature cited in the description**

- **WILSON, G. LEE et al.** Nuclear import of plasmid DNA in digitonin-permeabilized cells requires both cytoplasmic factors and specific DNA sequences.. *Journal of Biological Chemistry*, 1999, vol. 274 (31), 22025-22032

- **DEAN et al.** Sequence requirements for plasmid nuclear import. *Exp Cell Res*, 1999
- **MESIKA et al.** A regulated, NFkappaB-assisted import of plasmid DNA into mammalian cell nuclei.. *Mol Ther.*, 2001